# EUROPÄISCHE PATENTSCHRIFT

(11) **EP 2 231 645 B1**
(45) Veröffentlichungstag und Bekanntmachung des Hinweises auf die Patenterteilung: **03.10.2012**
(21) Anmeldenummer: 08858294.5
(22) Anmeldetag: 05.12.2008
(51) Int. Cl.: C07D 401/14, A61K 31/496, A61K 31/4545, A61P 3/10, A61P 7/02, A61P 9/00, A61P 25/00

(54) **5-Halogen-substitutierte Oxindol-Derivate und ihre Verwendung zur Herstellung eines Medikaments zur Behandlung von Vasopressin-abhängigen Erkrankungen**
5-Halogen-substituted oxindole derivatives and their use in the manufacture of a medicament for the treatment of vasopressin-dependent diseases
Dérivés oxindoliques substitués par halogène en position 5 et leur utilisation pour la préparation d'un medicament pour le traitement de maladies liées à la vasopressine

(30) Priorität: 07.12.2007 US 12234
(43) Veröffentlichungstag der Anmeldung: 29.09.2010
(73) Patentinhaber: Abbott GmbH & Co. KG, 65205 Wiesbaden (DE)
(72) Erfinder: BRAJE, Wilfried, 67061 Ludwigshafen (DE); OOST, Thorsten, 88400 Biberach An Der Riss (DE); NETZ, Astrid, 67061 Ludwigshafen (DE); WERNET, Wolfgang, verstorben (DE); UNGER, Liliane, 67061 Ludwigshafen (DE); HORNBERGER, Wilfried, 67061 Ludwigshafen (DE); LUBISCH, Wilfried, 69118 Heidelberg (DE)
(74) Vertreter: Reitstötter - Kinzebach
(86) Internationale Anmeldenummer: PCT/EP2008/066934
(87) Internationale Veröffentlichungsnummer: WO 2009/071689

(56) Entgegenhaltungen:
- WO-A-2005/030755
- WO-A-2006/005609
- WO-A-2008/080970
- WO-A-2008/080971
- WO-A-2008/080972
- WO-A-2008/080973

## Beschreibung

Die vorliegende Erfindung betrifft neue substituierte Oxindol-Derivate, diese enthaltende pharmazeutische Mittel und ihre Verwendung zur Herstellung eines Medicament zur Behandlung von Vasopressin-abhängigen Erkrankungen.

Vasopressin ist ein endogenes Hormon, das verschiedenste Wirkungen an Organen und Gewebe ausübt. Bei verschiedenen Krankheitszuständen, wie zum Beispiel Herzinsuffizienz und Bluthochdruck, vermutet man, dass das Vasopressin-System eine Rolle spielt. Derzeit sind drei Rezeptoren (V1a, V1b bzw. V3 und V2) bekannt, über die Vasopressin seine zahlreichen Wirkungen vermittelt. Daher werden Antagonisten dieser Rezeptoren als mögliche neue therapeutische Ansätze zur Behandlung von Krankheiten untersucht (M. Thibonnier, Exp.Opin. Invest. Drugs 1998, 7(5), 729-740).

Vorliegend werden neue substituierte Oxindole beschrieben, die in der 1-Position eine Phenylsulfonyl-Gruppe tragen. 1-Phenylsulfonyl-1,3-dihydro-2H-indol-2-one wurden bereits als Liganden der Vasopressin-Rezeptoren beschrieben. Auch die WO 93/15051, WO 95/18105, WO 98/25901, WO 01/55130, WO 01/55134, WO 01/164668 und WO 01/98295 beschreiben Derivate, die in der 1-Position des Oxindolgerüsts Arylsulfonlygruppen tragen. Diese Verbindungen unterscheiden sich von den erfindungsgemäßen Verbindungen wesentlich durch die Substituenten in der 3-Position.

So werden in der WO 93/15051 und WO 98/25901 1-Phenylsulfonyl-1,3-dihydro-2H-indol-2-one als Liganden der Vasopressinrezeptoren beschrieben, bei denen das Oxindol-Gerüst in der 3-Position durch zwei Alkylreste substituiert ist, die gemeinsam auch einen Cycloalkylrest (Spiroverknüpfung) bilden können. Als Alternative kann der Spiroring Heteroatome, wie Sauerstoff und Stickstoff (wahlweise mit Substituenten), enthalten.

Die WO 95/18105 beschreibt 1-Phenylsulfonyl-1,3-dihydro-2H-indol-2-one als Liganden der Vasopressinrezeptoren, die ein Stickstoffatom in der 3-Position besitzen. Zusätzlich sind in der 3-Position Reste gebunden, die unter gegebenenfalls substituierten Alkyl-, Cycloalkyl-, Phenyl- oder Benzylresten ausgewählt sind.

In der WO 03/008407 werden 1-Phenylsulfonyloxindole beschrieben, in denen in der 3-Position Pyridylpiperazine über eine Harnstoff-, Carbamat- oder 2-Oxoethyl-Gruppe am Oxindol gebunden sind.

In der WO 2005/030755 wird als Beispiel 105 4-(1-Methy)-piperidin-4-yl)-piperazin-1-carbonsäure-5-chlor-1-(2,4-dimethoxy-phenylsulfonyl)-3-(2-methoxy-pyridin-3-yl)-2-oxo-2,3-dihydro-1H-indol-3-yl-ester beschrieben.

In der WO 2006/005609 wird als Beispiel 66 das Dihydrochlorid von 4-(1-Methyl-piperidin-4-yl)-piperazin-1-carbonsäure [5-chlor-1-(2,4-dimethoxy-phenylsulfonyl)-3-(2-ethoxyphenyl)-2-oxo-2,3-dihydro-1H-indol-3-yl]-amid beschrieben.

Neben der Bindungsaffinität zum Vasopressin V1b Rezeptor können weitere Eigenschaften vorteilhaft für die Behandlung und/oder Prophylaxe von Vasopressin-abhängigen Erkrankungen sein, wie beispielsweise:
1.) eine Selektivität zum Vasopressin V1 b Rezeptor im Vergleich zum Vasopressin V1a Rezeptor, d.h. der Quotient aus der Bindungsaffinität zum V1a Rezeptor (Ki(V1a) (bestimmt in der Einheit "Nanomolar (nM)") und der Bindungsaffinität zum V1b Rezeptor (Ki(V1b)) (bestimmt in der Einheit "Nanomolar (nM)"). Je größer der Quotient Ki(V1a)/Ki(V1b) ist, desto größer ist die V1b-Selektivität;
2.) eine Selektivität zum Vasopressin V1b Rezeptor im Vergleich zum Vasopressin V2 Rezeptor, d.h. der Quotient aus der Bindungsaffinität zum V2 Rezeptor (Ki(V2) (bestimmt in der Einheit "Nanomolar (nM)") und der Bindungsaffinität zum V1b Rezeptor (Ki(V1b)) (bestimmt in der Einheit "Nanomolar (nM)"). Je größer der Quotient Ki(V2)/Ki(V1 b) ist, desto größer ist die V1b-Selektivität;
3.) eine Selektivität zum Vasopressin V1 b Rezeptor im Vergleich zum Oxytozin OT Rezeptor, d.h. der Quotient aus der Bindungsaffinität zum OT Rezeptor (Ki(OT) (bestimmt in der Einheit "Nanomolar (nM)") und der Bindungsaffinität zum V1 b Rezeptor (Ki(V1b)) (bestimmt in der Einheit "Nanomolar (nM)"). Je größer der Quotient Ki(OT)/Ki(V1 b) ist, desto größer ist die V1b-Selektivität.
4.) die metabolische Stabilität, beispielsweise bestimmt anhand der in-vitro ermittelten Halbwertszeiten in Lebermikrosomen von verschiedenen Spezies (z.B. Ratte oder Mensch);
5.) keine oder nur geringe Inhibierung von Cytochrom P450 (CYP) Enzymen: Cytochrom P450 (CYP) ist die Bezeichnung für eine Superfamilie von Hämproteinen mit enzymatischer Aktivität (Oxidase). Besondere Bedeutung besitzen sie auch für den Abbau (Metabolismus) von Fremdstoffen wie Pharmaka oder Xenobiotika in Säugetierorganismen. Die wichtigsten Vertreter der Typen und Untertypen von CYP im menschlichen Organismus sind: CYP 1A2, CYP 2C9, CYP 2D6 und CYP 3A4. Bei gleichzeitiger Anwendung von CYP 3A4-Hemmstoffen (z.B. Grapefruitsaft, Cimetidin, Erythromycin) und Arzneistoffen, die über dieses Enzymsystem abgebaut werden und somit um die gleiche Bindungsstelle am Enzym konkurrieren, kann deren Abbau verlangsamt und dadurch Wirkungen und Nebenwirkungen des verabreichten Arzneistoffs unerwünscht verstärkt werden;
6.) eine geeignete Wasserlöslichkeit (in mg/ml);
7.) eine geeignete Pharmakokinetik (zeitlicher Verlauf der Konzentration der erfindungsgemässen Verbindung im Plasma bzw. in Geweben, zum Beispiel Gehirn). Die Pharmakokinetik kann durch die folgenden Parameter beschrieben werden: Halbwertszeit (in h), Verteilungsvolumen (in I·kg⁻¹ Plasma-Clearance (in I·h⁻¹·kg⁻¹), AUC ("area under the curve", Fläche unter der Konzentrations-Zeit-Kurve, in ng·h·I⁻¹), orale Bioverfügbarkeit (das Dosis-normalisierte Verhältnis von AUC nach oraler Gabe und AUC nach intravenöser Gabe), das sog. Brain-Plasma-Ratio (das Verhältnis von AUC im Hirngewebe und AUC im Plasma);
8.) keine oder nur geringe Blockade des hERG-Kanals: Verbindungen, die den hERG-Kanal blockieren, können eine Verlängerung des QT-Intervalls verursachen und dadurch zu ernsten Herz-Rhythmus-Störungen führen (zum Beispiel sogenannte "torsade de pointes"). Mittels eines in der Literatur beschriebenen Verdrängungsassays mit radioaktiv markiertem Dofetilid (G. J. Diaz et al., Journal of Pharmacological and Toxicological Methods, 50 (2004), 187-199) kann das Potential von Verbindungen, den hERG-Kanal zu blockieren, bestimmt werden. Je geringer der IC50 in diesem "dofetilide assay", desto wahrscheinlicher ist eine potente hERG-Blockade. Darüber hinaus kann die Blockade des hERG-Kanals durch elektrophysiologische Experimente an Zellen, die mit dem hERG-Kanal transfiziert wurden, durch sogenanntes "whole-cell patch clamping" gemessen werden (G. J. Diaz et al., Journal of Pharmacological and Toxicological Methods, 50 (2004), 187-199).

Aufgabe der vorliegenden Erfindung war es, Verbindungen zu Anwendung in einem verfahren luidie Behandlung oder Prophylaxe von verschiedenen Vasopressin-abhängigen Krankheiten zur Verfügung zu stellen. Die Verbindungen sollten eine hohe Aktivität und Selektivität aufweisen, vor allem eine hohe Affinität und Selektivität gegebenüber dem Vasopressin V1b Rezeptor. Zusätzlich sollte die erfindungsgemäße Substanz einen oder mehrere der vorstehend genannten Vorteile 1.) bis 8.) besitzen.

Die Aufgabe wird durch Verbindungen der Formel I gelöst 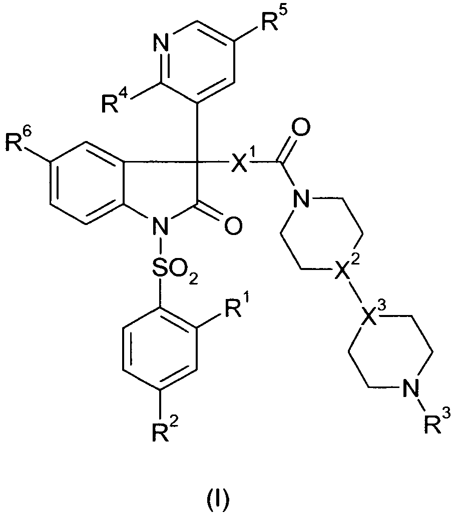 worin
- R¹: für Wasserstoff, Methoxy oder Ethoxy steht;
- R²: für Wasserstoff oder Methoxy steht;
- R³: für Wasserstoff, Methyl, Ethyl, n-Propyl oder Isopropyl steht;
- R⁴: für Ethoxy oder Isopropoxy steht;
- R⁵: für H oder Methyl steht;
- R⁶: für Cl oder F steht;
- X¹: für O, NH oder CH₂ steht; und
- X² und X³: für N oder CH stehen, unter der Maßgabe, dass X² und X³ nicht gleichzeitig für N stehen;
sowie deren pharmazeutisch verträglichen Salze

Dementsprechend betrifft die vorliegende Erfindung Verbindungen der Formel I (im Folgenden auch "Verbindungen I"), sowie die pharmazeutisch verträgliche Salze der Verbindungen I.

Die pharmazeutisch verträglichen Salze von Verbindungen der Formel I, die auch als physiologisch verträgliche Salze bezeichnet werden, sind in der Regel durch Umsetzung der freien Base der erfindungsgemäßen Verbindungen I (d.h. der Verbindungen I gemäß Strukturformel I) mit geeigneten Säuren erhältlich. Geeignete Säuren sind beispielsweise aufgeführt in "Fortschritte der Arzneimittelforschung", 1966, Birkhäuser Verlag, Bd.10, S.224-285. Darunter fallen beispielsweise Salzsäure, Zitronensäure, Weinsäure, Milchsäure, Phosphorsäure, Methansulfonsäure, Essigsäure, Ameisensäure, Maleinsäure und Fumarsäure.

C₁-C₄-Alkyl steht im Rahmen der vorliegenden Erfindung für einen linearen oder verzweigten Alkylrest mit 1 bis 4 Kohlenstoffatomen, wie Methyl, Ethyl, n-Propyl, Isopropyl, n-Butyl, sek-Butyl, Isobutyl oder tert-Butyl.

C₁-C₃-Alkoxy steht im Rahmen der vorliegenden Erfindung für einen über ein Sauerstoffatom gebundenen linearen oder verzweigten Alkylrest mit 1 bis 3 Kohlenstoffatomen. Beispiele sind Methoxy, Ethoxy, n-Propoxy und Isopropoxy.

Die erfindungsgemäßen Verbindungen der Formel I und ihre pharmakologisch verträglichen Salze können auch in Form von Solvaten oder Hydraten vorliegen. Unter Solvaten versteht man im Rahmen der vorliegenden Erfindung kristalline Formen der Verbindungen I bzw. ihrer pharmazeutisch verträglichen Salze , die im Kristallgitter Lösungsmittelmoleküle eingebaut enthalten. Vorzugsweise sind die Lösungsmittelmoleküle in stöchiometrischen Verhältnissen eingebaut. Hydrate sind eine Spezialform der Solvate; das Lösungsmittel ist hier Wasser.

Die nachstehend gemachten Angaben zu geeigneten und bevorzugten Merkmalen der Erfindung, insbesondere zu den Resten R¹, R², R³, R⁴, R⁵, R⁶, X¹, X² und X³ der Verbindung I, aber auch zu den Merkmalen des erfindungsgemäßen Verfahrens und der erfindungsgemäßen Verwendung gelten sowohl für sich allein genommen als auch vorzugsweise in jeder möglichen Kombination miteinander.

Die Verbindungen I werden bevorzugt in Form der freien Base (d.h. gemäß der Strukturformel I) oder in Form ihrer Säureadditionssalze bereitgestellt.

In einer bevorzugten Ausführungsform steht R¹ für Wasserstoff oder Methoxy.

In einer besonders bevorzugten Ausführungsform stehen R¹ und R² für Methoxy.

In einer bevorzugten Ausführungsform steht R³ für Wasserstoff, Methyl oder Ethyl, insbesondere für Wasserstoff oder Methyl und speziell für Methyl.

In einer bevorzugten Ausführungsform steht R⁴ für Ethoxy und R⁵ steht für H.

In einer alternativ bevorzugten Ausführungsform steht R⁴ für Ethoxy und R⁵ steht für Methyl.

In einer alternativ bevorzugten Ausführungsform steht R⁴ für Isopropoxy und R⁵ steht für H.

Besonders bevorzugt steht R⁴ für Ethoxy und R⁵ steht für H.

In einer bevorzugten Ausführungsform steht R⁶ für Cl.

In einer alternativ bevorzugten Ausführungsform steht R⁶ für F.

Besonders bevorzugt steht R⁶ für Cl.

In einer bevorzugten Ausführungsform steht X¹ für NH.

In einer alternativ bevorzugten Ausführungsform steht X¹ für O.

In einer alternativ bevorzugten Ausführungsform steht X¹ für CH₂.

Besonders bevorzugt steht X¹ für NH oder CH₂ und insbesondere für NH.

In einer bevorzugten Ausführungsform steht eine der Variablen X², X³ für N und die andere für CH.

In einer besonders bevorzugten Ausführungsform steht dabei X² für N und X³ steht für CH.

In einer alternativ besonders bevorzugten Ausführungsform steht X² für CH und X³ steht für N.

In einer alternativ bevorzugten Ausführungsform stehen beide Variablen X², X³ für CH.

Ein bevorzugter Gegenstand der Erfindung sind Verbindungen der Formel I, worin
- R¹: Wasserstoff oder Methoxy ist;
- R²: Wasserstoff oder Methoxy ist;
- R³: Wasserstoff, Methyl Ethyl, n-Propyl oder Isopropyl; vorzugsweise Wasserstoff, Methyl oder Ethyl ist;
- R⁴: Ethoxy ist;
- R⁵: Wasserstoff ist;
- R⁶: Cl ist;
- X¹: NH, O oder CH₂ ist;
- X²: N oder CH ist;
- X³: N oder CH ist;
wobei X² und X³ nicht gleichzeitig für N stehen;
sowie die pharmazeutisch verträglichen Salze davon.

Ein besonders bevorzugter Gegenstand der Erfindung sind Verbindungen der Formel I, worin
- R¹: Wasserstoff oder Methoxy ist;
- R²: Wasserstoff oder Methoxy ist;
- R³: Methyl oder Ethyl ist;
- R⁴: Ethoxy ist;
- R⁵: Wasserstoff ist;
- R⁶: Cl ist;
- X¹: NH, O oder CH₂ ist;
- X²: N oder CH ist;
- X³: N oder CH ist;
wobei X² und X³ nicht gleichzeitig für N stehen;
sowie die pharmazeutisch verträglichen Salze davon.

Ein stärker bevorzugter Gegenstand der Erfindung sind Verbindungen der Formel I, worin
- R¹: Methoxy ist;
- R²: Methoxy ist;
- R³: Methyl oder Ethyl ist;
- R⁴: Ethoxy ist;
- R⁵: Wasserstoff ist;
- R⁶: Cl ist;
- X¹: NH, O oder CH₂ ist;
- X² -: N oder CH ist;
- X³: N oder CH ist;
wobei X² und X³ nicht gleichzeitig für N stehen;
sowie die pharmazeutisch verträglichen Salze davon.

Ein noch stärker bevorzugter Gegenstand der Erfindung sind Verbindungen der Formel I, worin
- R¹: Methoxy ist;
- R²: Methoxy ist;
- R³: Methyl oder Ethyl ist;
- R⁴: Ethoxy ist;
- R⁵: Wasserstoff ist;
- R⁶: Cl ist;
- X¹: NH ist;
- X²: N oder CH ist;
- X³: N oder CH ist;
wobei X² und X³ nicht gleichzeitig für N stehen;
sowie die pharmazeutisch verträglichen Salze davon.

Ein alternativ noch stärker bevorzugter Gegenstand der Erfindung sind Verbindungen der Formel I worin
- R¹: Methoxy ist;
- R²: Methoxy ist;
- R³: Methyl oder Ethyl ist;
- R⁴: Ethoxy ist;
- R⁵: Wasserstoff ist;
- R⁶: Cl ist;
- X¹: CH₂ ist;
- X²: N oder CH ist;
- X³: N oder CH ist;
wobei X² und X³ nicht gleichzeitig für N stehen;
sowie die pharmazeutisch verträglichen Salze davon.

Insbesondere sind Verbindungen der Formel I Gegenstand der Erfindung, worin
- R¹: Methoxy ist;
- R²: Methoxy ist;
- R³: Methyl oder Ethyl ist;
- R⁴: Ethoxy ist;
- R⁵: Wasserstoff ist;
- R⁶: Cl ist;
- X¹: NH ist;
- X²: N ist;
- X³: CH ist;
sowie die pharmazeutisch verträglichen Salze davon.

Insbesondere sind auch Verbindungen der Formel I Gegenstand der Erfindung, worin
- R¹: Methoxy ist;
- R²: Methoxy ist;
- R³: Methyl oder Ethyl ist;
- R⁴: Ethoxy ist;
- R⁵: Wasserstoff ist;
- R⁶: Cl ist;
- X¹: NH ist;
- X²: CH ist;
- X³: N ist;
sowie die pharmazeutisch verträglichen Salze davon.

Insbesondere sind auch Verbindungen der Formel I Gegenstand der Erfindung, worin
- R¹: Methoxy ist;
- R²: Methoxy ist;
- R³: Methyl oder Ethyl ist;
- R⁴: Ethoxy ist;
- R⁵: Wasserstoff ist;
- R⁶: Cl ist;
- X¹: NH ist;
- X²: CH ist;
- X³: CH ist;
sowie die pharmazeutisch verträglichen Salze davon.

Ein alternativ bevorzugter Gegenstand der Erfindung sind Verbindungen der Formel I, worin
- R¹: Wasserstoff oder Methoxy ist;
- R²: Wasserstoff oder Methoxy ist;
- R³: Wasserstoff, Methyl Ethyl, n-Propyl oder Isopropyl; vorzugsweise Wasserstoff, Methyl oder Ethyl ist;
- R⁴: Ethoxy ist;
- R⁵: Methyl ist;
- R⁶: Cl ist;
- X¹: NH, O oder CH₂ ist;
- X²: N oder CH ist;
- X³: N oder CH ist;
wobei X² und X³ nicht gleichzeitig für N stehen;
sowie die pharmazeutisch verträglichen Salze davon.

Ein alternativ besonders bevorzugter Gegenstand der Erfindung sind Verbindungen der Formel I, worin
- R¹: Wasserstoff oder Methoxy ist;
- R²: Wasserstoff oder Methoxy ist;
- R³: Methyl oder Ethyl ist;
- R⁴: Ethoxy ist;
- R⁵: Methyl ist;
- R⁶: Cl ist;
- X¹: NH, O oder CH₂ ist;
- X²: N oder CH ist;
- X³: N oder CH ist;
wobei X² und X³ nicht gleichzeitig für N stehen;
sowie die pharmazeutisch verträglichen Salze davon.

Ein alternativ stärker bevorzugter Gegenstand der Erfindung sind Verbindungen der Formel I, worin
- R¹: Methoxy ist;
- R²: Methoxy ist;
- R³: Methyl oder Ethyl ist;
- R⁴: Ethoxy ist;
- R⁵: Methyl ist;
- R⁶: Cl ist;
- X¹: NH, O oder CH₂ ist;
- X²: N oder CH ist;
- X³: N oder CH ist;
wobei X² und X³ nicht gleichzeitig für N stehen;
sowie die pharmazeutisch verträglichen Salze davon.

Ein alternativ noch stärker bevorzugter Gegenstand der Erfindung sind Verbindungen der Formel I, worin
- R¹: Methoxy ist;
- R²: Methoxy ist;
- R³: Methyl oder Ethyl ist;
- R⁴: Ethoxy ist;
- R⁵: Methyl ist;
- R⁶: Cl ist;
- X¹: NH ist;
- X²: N oder CH ist;
- X³: N oder CH ist;
wobei X² und X³ nicht gleichzeitig für N stehen;
sowie die pharmazeutisch verträglichen Salze davon.

Ein alternativ noch stärker bevorzugter Gegenstand der Erfindung sind Verbindungen der Formel I, worin
- R¹: Methoxy ist;
- R²: Methoxy ist;
- R³: Methyl oder Ethyl ist;
- R⁴: Ethoxy ist;
- R⁵: Methyl ist;
- R⁶: Cl ist;
- X¹: CH₂ ist;
- X²: N oder CH ist;
- X³: N oder CH ist;
wobei X² und X³ nicht gleichzeitig für N stehen;
sowie die pharmazeutisch verträglichen Salze davon.

Insbesondere sind Verbindungen der Formel I Gegenstand der Erfindung, worin
- R¹: Methoxy ist;
- R²: Methoxy ist;
- R³: Methyl oder Ethyl ist;
- R⁴: Ethoxy ist;
- R⁵: Methyl ist;
- R⁶: Cl ist;
- X¹: NH ist;
- X²: N ist;
- X³: CH ist;
sowie die pharmazeutisch verträglichen Salze davon.

Insbesondere sind auch Verbindungen der Formel I Gegenstand der Erfindung, worin
- R¹: Methoxy ist;
- R²: Methoxy ist;
- R³: Methyl oder Ethyl ist;
- R⁴: Ethoxy ist;
- R⁵: Methyl ist;
- R⁶: Cl ist;
- X¹: NH ist;
- X²: CH ist;
- X³: N ist;
sowie die pharmazeutisch verträglichen Salze davon.

Insbesondere sind auch Verbindungen der Formel I Gegenstand der Erfindung, worin
- R¹: Methoxy ist;
- R²: Methoxy ist;
- R³: Methyl oder Ethyl ist;
- R⁴: Ethoxy ist;
- R⁵: Methyl ist;
- R⁶: Cl ist;
- X¹: NH ist;
- X²: CH ist;
- X³: CH ist;
sowie die pharmazeutisch verträglichen Salze davon.

Speziell bevorzugt ist die Verbindung I, worin
- R¹: Methoxy ist;
- R²: Methoxy ist;
- R³: Methyl ist;
- R⁴: Ethoxy ist;
- R⁵: Wasserstoff ist ist;
- R⁶: Cl ist;
- X¹: NH ist;
- X²: N ist;
- X³: CH ist;
sowie die pharmazeutisch verträglichen Salze davon.

Beispiele für bevorzugte Ausführungsform der vorliegenden Erfindung sind Verbindungen der Formel I.1 bis 1.18 sowie die pharmazeutisch verträglichen Salze davon, worin die Reste X², X³, R¹, R² und R³ jeweils die in der folgenden Tabelle 1 pro Zeile genannten Bedeutungen annehmen.

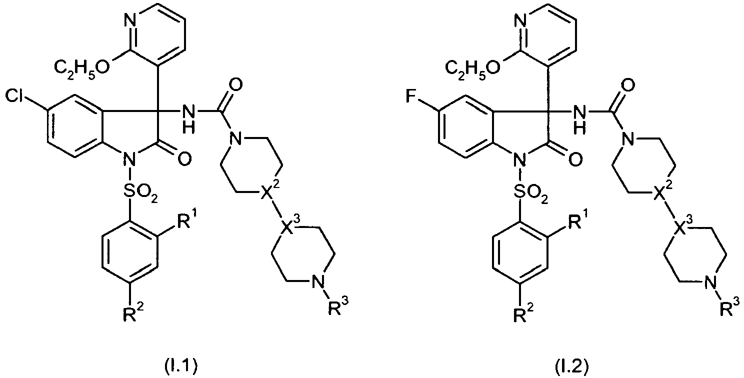

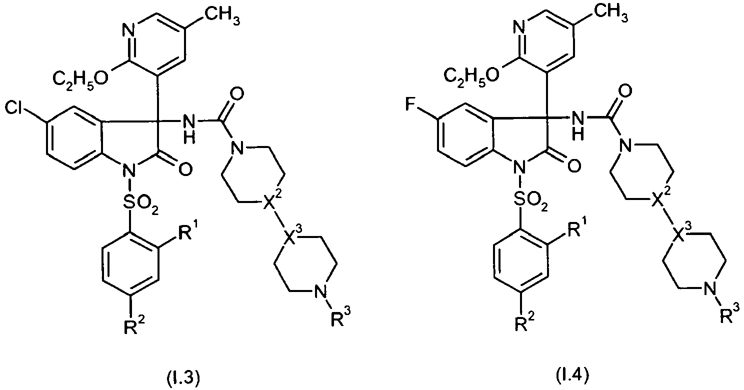

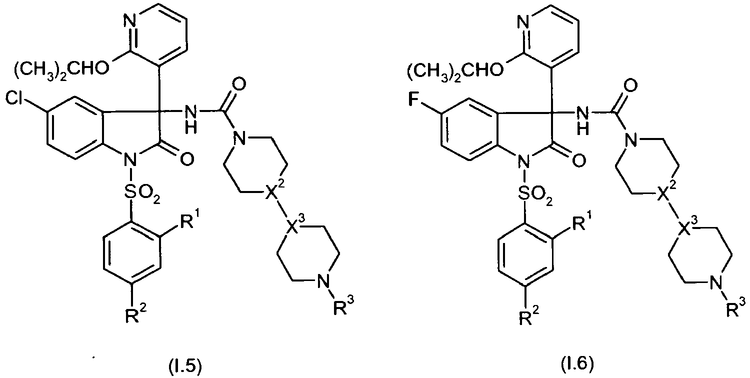

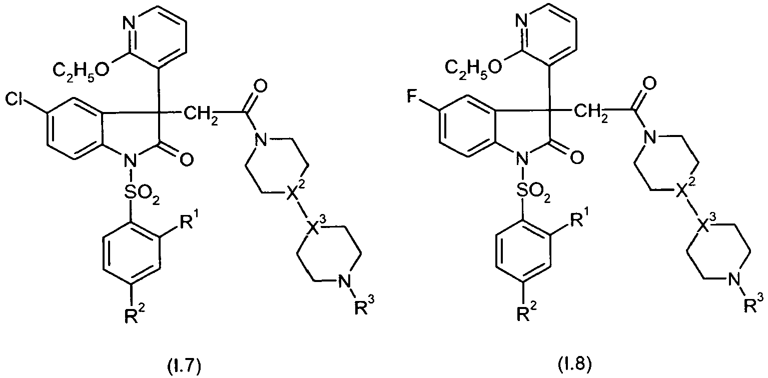

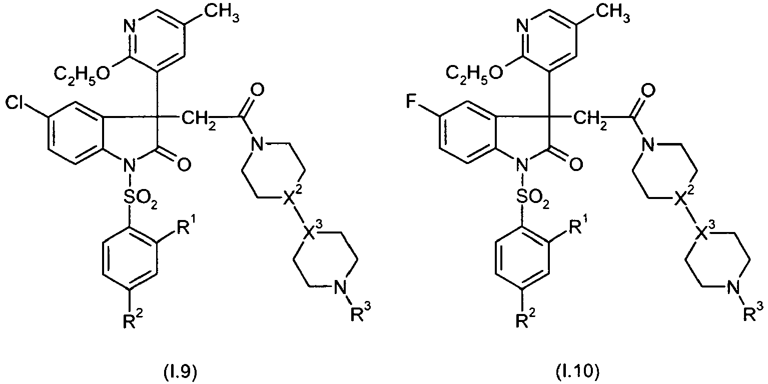

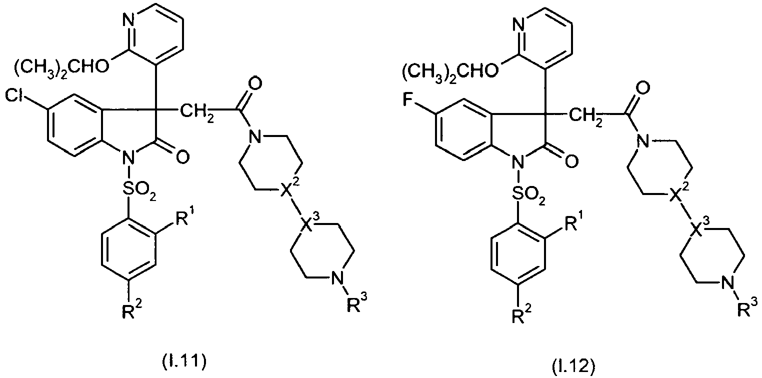

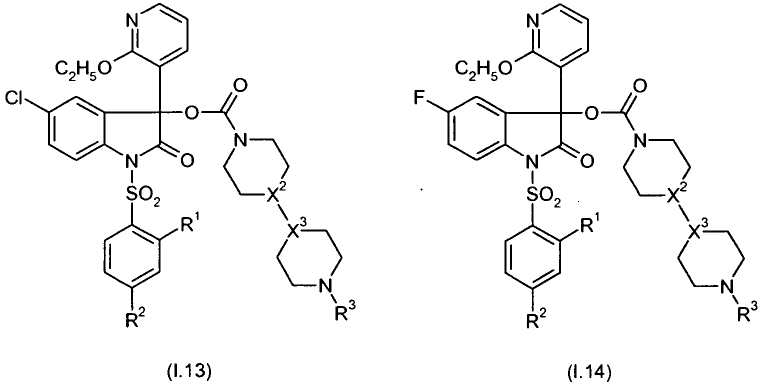

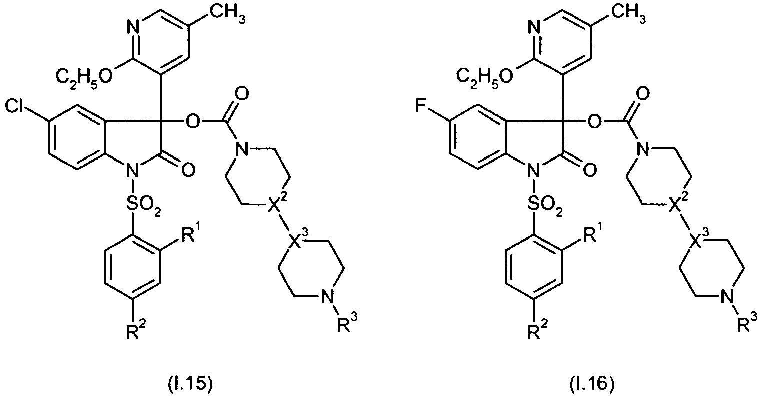

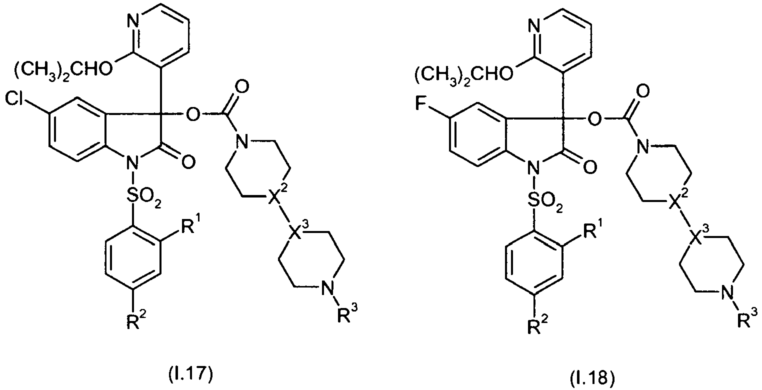

**Tabelle 1:**

| **Beispiel Nr.** | **X²** | **X³** | **R¹** | **R²** | **R³** |
|---|---|---|---|---|---|
| A-1. | N | CH | Methoxy | Methoxy | Methyl |
| A-2. | N | CH | Methoxy | H | Methyl |
| A-3. | N | CH | Ethoxy | H | Methyl |
| A-4. | N | CH | H | H | Methyl |
| A-5. | N | CH | H | Methoxy | Methyl |
| A-6. | N | CH | Ethoxy | Methoxy | Methyl |
| A-7. | N | CH | Methoxy | Methoxy | Ethyl |
| A-8. | N | CH | Methoxy | H | Ethyl |
| A-9. | N | CH | Ethoxy | H | Ethyl |
| A-10. | N | CH | H | H | Ethyl |
| A-11. | N | CH | H | Methoxy | Ethyl |
| A-12. | N | CH | Ethoxy | Methoxy | Ethyl |
| A-13. | N | CH | Methoxy | Methoxy | n-Propyl |
| A-14. | N | CH | Methoxy | H | n-Propyl |
| A-15. | N | CH | Ethoxy | H | n-Propyl |
| A-16. | N | CH | H | H | n-Propyl |
| A-17. | N | CH | H | Methoxy | n-Propyl |
| A-18. | N | CH | Ethoxy | Methoxy | n-Propyl |
| A-19. | N | CH | Methoxy | Methoxy | Isopropyl |
| A-20. | N | CH | Methoxy | H | Isopropyl |
| A-21. | N | CH | Ethoxy | H | Isopropyl |
| A-22. | N | CH | H | H | Isopropyl |
| A-23. | N | CH | H | Methoxy | Isopropyl |
| A-24. | N | CH | Ethoxy | Methoxy | Isopropyl |
| A-25. | N | CH | Methoxy | Methoxy | H |
| A-26. | N | CH | Methoxy | H | H |
| A-27. | N | CH | Ethoxy | H | H |
| A-28. | N | CH | H | H | H |
| A-29. | N | CH | H | Methoxy | H |
| A-30. | N | CH | Ethoxy | Methoxy | H |
| A-31. | CH | N | Methoxy | Methoxy | Methyl |
| A-32. | CH | N | Methoxy | H | Methyl |
| A-33. | CH | N | Ethoxy | H | Methyl |
| A-34. | CH | N | H | H | Methyl |
| A-35. | CH | N | H | Methoxy | Methyl |
| A-36. | CH | N | Ethoxy | Methoxy | Methyl |
| A-37. | CH | N | Methoxy | Methoxy | Ethyl |
| A-38. | CH | N | Methoxy | H | Ethyl |
| A-39. | CH | N | Ethoxy | H | Ethyl |
| A-40. | CH | N | H | H | Ethyl |
| A-41. | CH | N | H | Methoxy | Ethyl |
| A-42. | CH | N | Ethoxy | Methoxy | Ethyl |
| A-43. | CH | N | Methoxy | Methoxy | n-Propyl |
| A-44. | CH | N | Methoxy | H | n-Propyl |
| A-45. | CH | N | Ethoxy | H | n-Propyl |
| A-46. | CH | N | H | H | n-Propyl |
| A-47. | CH | N | H | Methoxy | n-Propyl |
| A-48. | CH | N | Ethoxy | Methoxy | n-Propyl |
| A-49. | CH | N | Methoxy | Methoxy | Isopropyl |
| A-50. | CH | N | Methoxy | H | Isopropyl |
| A-51. | CH | N | Ethoxy | H | Isopropyl |
| A-52. | CH | N | H | H | Isopropyl |
| A-53. | CH | N | H | Methoxy | Isopropyl |
| A-54. | CH | N | Ethoxy | Methoxy | Isopropyl |
| A-55. | CH | N | Methoxy | Methoxy | H |
| A-56. | CH | N | Methoxy | H | H |
| A-57. | CH | N | Ethoxy | H | H |
| A-58. | CH | N | H | H | H |
| A-59. | CH | N | H | Methoxy | H |
| A-60. | CH | N | Ethoxy | Methoxy | H |
| A-61. | CH | CH | Methoxy | Methoxy | Methyl |
| A-62. | CH | CH | Methoxy | H | Methyl |
| A-63. | CH | CH | Ethoxy | H | Methyl |
| A-64. | CH | CH | H | H | Methyl |
| A-65. | CH | CH | H | Methoxy | Methyl |
| A-66. | CH | CH | Ethoxy | Methoxy | Methyl |
| A-67. | CH | CH | Methoxy | Methoxy | Ethyl |
| A-68. | CH | CH | Methoxy | H | Ethyl |
| A-69. | CH | CH | Ethoxy | H | Ethyl |
| A-70. | CH | CH | H | H | Ethyl |
| A-71. | CH | CH | H | Methoxy | Ethyl |
| A-72. | CH | CH | Ethoxy | Methoxy | Methyl |
| A-73. | CH | CH | Methoxy | Methoxy | n-Propyl |
| A-74. | CH | CH | Methoxy | H | n-Propyl |
| A-75. | CH | CH | Ethoxy | H | n-Propyl |
| A-76. | CH | CH | H | H | n-Propyl |
| A-77. | CH | CH | H | Methoxy | n-Propyl |
| A-78. | CH | CH | Ethoxy | Methoxy | n-Propyl |
| A-79. | CH | CH | Methoxy | Methoxy | Isopropyl |
| A-80. | CH | CH | Methoxy | H | Isopropyl |
| A-81. | CH | CH | Ethoxy | H | Isopropyl |
| A-82. | CH | CH | H | H | Isopropyl |
| A-83. | CH | CH | H | Methoxy | Isopropyl |
| A-84. | CH | CH | Ethoxy | Methoxy | Isopropyl |
| A-85. | CH | CH | Methoxy | Methoxy | H |
| A-86. | CH | CH | Methoxy | H | H |
| A-87. | CH | CH | Ethoxy | H | H |
| A-88. | CH | CH | H | H | H |
| A-89. | CH | CH | H | Methoxy | H |
| A-90. | CH | CH | Ethoxy | Methoxy | H |

Unter den oben genannten Verbindungen I.1 bis 1.18 sind Verbindungen der Formeln 1.1, I.3, I.5, I.7, I.9, I.11, I.13, I.15 und 1.17, worin die Reste X², X³, R¹, R² und R³ jeweils die in der Tabelle 1 pro Zeile genannten Bedeutungen annehmen, bevorzugt. Hierunter bevorzugt sind wiederum die Verbindungen der Formeln I.1, I.3, I.5, I.7 und I.13, worin die Reste X², X³, R¹, R² und R³ jeweils die in der Tabelle 1 pro Zeile genannten Bedeutungen annehmen.

Besonders bevorzugt sind die Verbindungen der Formel 1.1, worin die Reste X², X³, R¹, R² und R³ jeweils die in der Tabelle 1 in den Zeilen A-1, A-2, A-4, A-7, A-31, A-32, A-34, A-37, A-38, A-40, A-61, A-67 und A-85, vorzugsweise die in den Zeilen A-1, A-2, A-4, A-7, A-31, A-37, A-38, A-61 und A-67, und insbesondere die in den Zeilen A-1, A-4, A-7, A-31, A-37, A-61 und A-67 genannten Bedeutungen annehmen.

Besonders bevorzugt sind auch die Verbindungen der Formel 1.3, worin die Reste X², X³, R¹, R² und R³ jeweils die in der Tabelle 1 in den Zeilen A-1, A-7, A-31 und A-37, und vorzugsweise die in den Zeilen A-1 und A-7 genannten Bedeutungen annehmen.

Besonders bevorzugt sind auch die Verbindungen der Formel 1.5, worin die Reste X², X³, R¹, R² und R³ jeweils die in der Tabelle 1 in den Zeilen A-1, A-7, A-31 und A-37, und vorzugsweise die in den Zeilen A-1 und A-7 genannten Bedeutungen annehmen.

Besonders bevorzugt sind auch die Verbindungen der Formel 1.7, worin die Reste X², X³, R¹, R² und R³ jeweils die in der Tabelle 1 in den Zeilen A-1, A-4, A-31, A-34, A-37und A-40, und vorzugsweise die in Zeile A-1 genannten Bedeutungen annehmen.

Besonders bevorzugt sind auch die Verbindungen der Formel 1.13, worin die Reste X², X³, R¹, R² und R³ jeweils die in der Tabelle 1 in den Zeilen A-1, A-2, A-4, A-31, A-32 und A-34, und vorzugsweise die in Zeile A-2 genannten Bedeutungen annehmen.

Unter diesen besonders bevorzugten Verbindungen sind wiederum die Verbindungen I.1, 1.3,1.5 und 1.7 bevorzugt, wobei die Reste X², X³, R¹, R² und R³ die zu den jeweiligen Verbindungen angegebenen Bedeutungen haben. Besonders bevorzugt sind hierunter Verbindungen der Formeln 1.1, 1.3 und 1.5 und insbesondere der Formeln 1.1 und 1.3, wobei Verbindungen der Formel I.1 noch stärker bevorzugt sind.

Die erfindungsgemäßen Verbindungen I weisen in der 3-Position des 2-Oxindolrings ein Chiralitätszentrum auf. Die erfindungsgemäßen Verbindungen können daher als ein 1:1-Gemisch von Enantiomeren (Racemat) oder als ein nicht-racemisches Gemisch von Enantiomeren, in dem eines der beiden Enantiomere, entweder das die Schwingungsebene von linear polarisiertem Licht nach links drehende (d.h. minusdrehende) Enantiomer (im Folgenden (-)-Enantiomer) oder das die Schwingungsebene von linear polarisiertem Licht nach rechts drehende (d.h. plusdrehende) Enantiomer (im Folgenden (+)-Enantiomer), angereichert ist, oder als im Wesentlichen enantiomerenreine Verbindungen, also als im Wesentlichen enantiomerenreines (-)-Enantiomer oder (+)-Enantiomer, vorliegen. Da bei den erfindungsgemäßen Verbindungen ein einziges Asymmetriezentrum und keine Chiralitätsachse/-ebene existiert, kann man ein nicht-racemisches Gemisch auch als ein Gemisch von Enantiomeren definieren, in welchem entweder das R- oder das S-Enantiomer überwiegt. Im Wesentlichen enantiomerenreine Verbindungen können dementsprechend auch als im Wesentlichen enantiomerenreines R-Enantiomer bzw. im Wesentlichen enantiomerenreines S-Enantiomer definiert werden.

Unter "im Wesentlichen enantiomerenreinen Verbindungen" versteht man im Rahmen der vorliegenden Erfindung solche Verbindungen, die einen Enantiomerenüberschuss (enantiomeric excess, ee; % ee = (R-S)/(R+S) x 100 bzw. (S-R)/(S+R) x 100) von wenigstens 80 % ee, vorzugsweise wenigsten 85 % ee, stärker bevorzugt wenigstens 90 % ee, noch stärker bevorzugt wenigstens 95 % ee und insbesondere wenigstens 98 % ee aufweisen.

In einer Ausführungsform der Erfindung liegen die erfindungsgemäßen Verbindungen als im Wesentlichen enantiomerenreine Verbindungen vor. Besonders bevorzugt sind Verbindungen, die einen Enantiomerenüberschuss von wenigstens 85 % ee, stärker bevorzugt von wenigstens 90 % ee, noch stärker bevorzugt von wenigstens 95 % ee und insbesondere von wenigstens 98 % ee aufweisen.

Gegenstand der Erfindung sind somit sowohl die reinen Enantiomere als auch deren Gemische, z.B. Gemische, in denen ein Enantiomer in angereichter Form vorliegt, aber auch die Racemate. Gegenstand der Erfindung sind auch die pharmazeutisch verträglichen Salze der reinen Enantiomere von Verbindungen I sowie die Enantiomerengemische in Form der pharmazeutisch verträglichen Salze von Verbindungen I.

Bevorzugte Ausführungsformen der Erfindung sind Verbindungen der Formel I, wie vorstehend ausgeführt, die dadurch gekennzeichnet sind, dass sie in optisch aktiver Form vorliegen und es sich jeweils um das die Schwingungsebene von polarisiertem Licht nach rechts drehende (d.h. das plusdrehende) Enantiomer der betreffenden Verbindung der Formel I in der Form der freien Base handelt, oder um ein pharmazeutisch verträgliches Salz davon. Die rechts- bzw. plusdrehenden Enantiomere der Verbindungen I werden im Folgenden auch als (+)-Enantiomere bezeichnet.

Besonders bevorzugt sind Verbindungen der allgemeinen Formel I und deren pharmazeutisch verträglichen Salze, wie vorstehend ausgeführt, worin das entsprechende (+)-Enantiomer in einer optischen Reinheit (enantiomeric excess, ee) von größer 50 % ee, besonders bevorzugt von wenigstens 80 % ee, stärker bevorzugt von wenigstens 90 % ee, noch stärker bevorzugt von wenigstens 95 % ee und insbesondere von wenigstens 98 % ee vorliegt.

Ebenfalls bevorzugte Ausführungsformen der Erfindung sind Verbindungen der allgemeinen Formel I, wie vorstehend ausgeführt, die dadurch gekennzeichnet sind, dass sie in optisch inaktiver Form, d.h. in Form des Racemats, vorliegen, oder in Form eines pharmazeutisch verträglichen Salzes des Racemats.

Die im Rahmen der vorliegenden Erfindung gemachten Angaben zur Drehrichtung des polarisierten Lichts beziehen sich vorzugsweise auf Vorzeichen [(+) oder (-)] wie sie in Chloroform als Lösungsmittel oder in Chloroform-haltigen Lösungsmittelgemischen, insbesondere in Chloroform, ermittelt werden.

Im Folgenden werden beispielhafte Synthesewege zur Herstellung der erfindungsgemäßen Oxindol-Derivate beschrieben.

Die Herstellung der erfindungsgemäßen Verbindungen kann unter Verwendung der in WO 2005/030755 und WO 2006/005609 beschriebenen Vorschriften zur Synthese analoger Verbindungen erfolgen und ist beispielhaft in den Syntheseschemata 1 bis 3 skizziert. In diesen Syntheseschemata besitzen die Variablen die gleichen Bedeutungen wie in der Formel I.

Die 3-Hydroxy-1,3-dihydroindol-2-one IV können durch Addition von metallierten Heterocyclen III an die 3-Ketogruppe der Isatine II erhalten werden. Die metallierten Heterocyclen, wie beispielsweise die entsprechenden Grignard- (Mg) oder Organyllithium-Verbindung, können in üblicher Weise aus Halogen- oder Kohlenwasserstoffverbindungen erhalten werden. Beispielhafte Vorschriften sind im Houben-Weyl, Methoden der Organischen Chemie, Bd. 13, 1-2, Kap. Mg- bzw. Li-Verbindungen, enthalten. Die Isatine II sind entweder kommerziell erhältlich oder wurden in Analogie zu in der Literatur beschriebenen Methoden hergestellt (Advances in Heterocyclic Chemistry, A.R. Katritzky and A.J. Boulton, Academic Press, New York, 1975, 18, 2-58; J. Brazil. Chem. Soc. 12, 273-324, 2001).

Die 3-Hydroxyoxindole IV können in die Verbindungen V überführt werden, welche eine Fluchtgruppe LG' in 3-Stellung tragen, wobei die Fluchtgruppe LG' eine übliche Abgangsgruppe ist, wie zum Beispiel Chlorid oder Bromid. Das Zwischenprodukt V mit zum Beispiel LG' = Chlor kann durch Behandlung des Alkohols IV mit Thionylchlorid in Gegenwart einer Base, wie zum Beispiel Pyridin, in einem geeigneten Lösungsmittel, wie zum Beispiel Dichlormethan, hergestellt werden.

Die Verbindungen V können anschließend mit Aminen, wie beispielsweise Ammoniak, in einer Substitutionsreaktion zu den Aminen VI umgesetzt werden. Die Verbindungen VI können anschließend durch Behandlung mit Sulfonsäurechloriden VII nach Deprotonierung mit einer starken Base, wie zum Beispiel Kalium-tert-butylat oder Natriumhydrid in DMF, in das sulfonylierte Produkt VIII überführt werden. Die eingesetzten Sulfonsäurechloride VII können entweder käuflich erworben oder nach bekannten Verfahren (zum Beispiel J. Med. Chem. 40, 1149 (1997)) hergestellt werden.

Die Herstellung der erfindungsgemäßen Verbindungen der allgemeinen Formel I, welche eine Harnstoff-Gruppe in der 3-Stellung tragen, kann wie in WO 2005/030755 und WO 2006/005609 beschrieben und in Syntheseschema 1 gezeigt in einem zweistufigen Verfahren erfolgen: Zuerst werden die Verbindungen VIII mit Chlorameisensäurephenylester in Gegenwart einer Base, wie zum Beispiel Pyridin, zu dem entsprechenden Phenylcarbamat IX umgesetzt.

Die anschließende Umsetzung mit Aminen X, gegebenenfalls bei erhöhter Temperatur und unter Zusatz von Hilfsbasen wie beispielsweise Triethylamin oder Diisopropylethylamin führt zu den erfindungsgemäßen Verbindungen der allgemeinen Formel (I) mit Harnstoff-Brücke (X¹ = NH). Die Amine X können entweder käuflich erworben oder nach Literatur-bekannten Methoden hergestellt werden. Die Darstellung der erfindungsgemäßen Verbindungen I mit R³ = H kann durch Verwendung entsprechender Boc-geschützter Amine X (R³ = Boc) erfolgen. Die Boc-Schutzgruppe kann anschließend entfernt werden, zum Beispiel durch Behandlung mit Trifluoressigsäure in Dichlormethan.

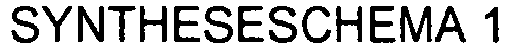

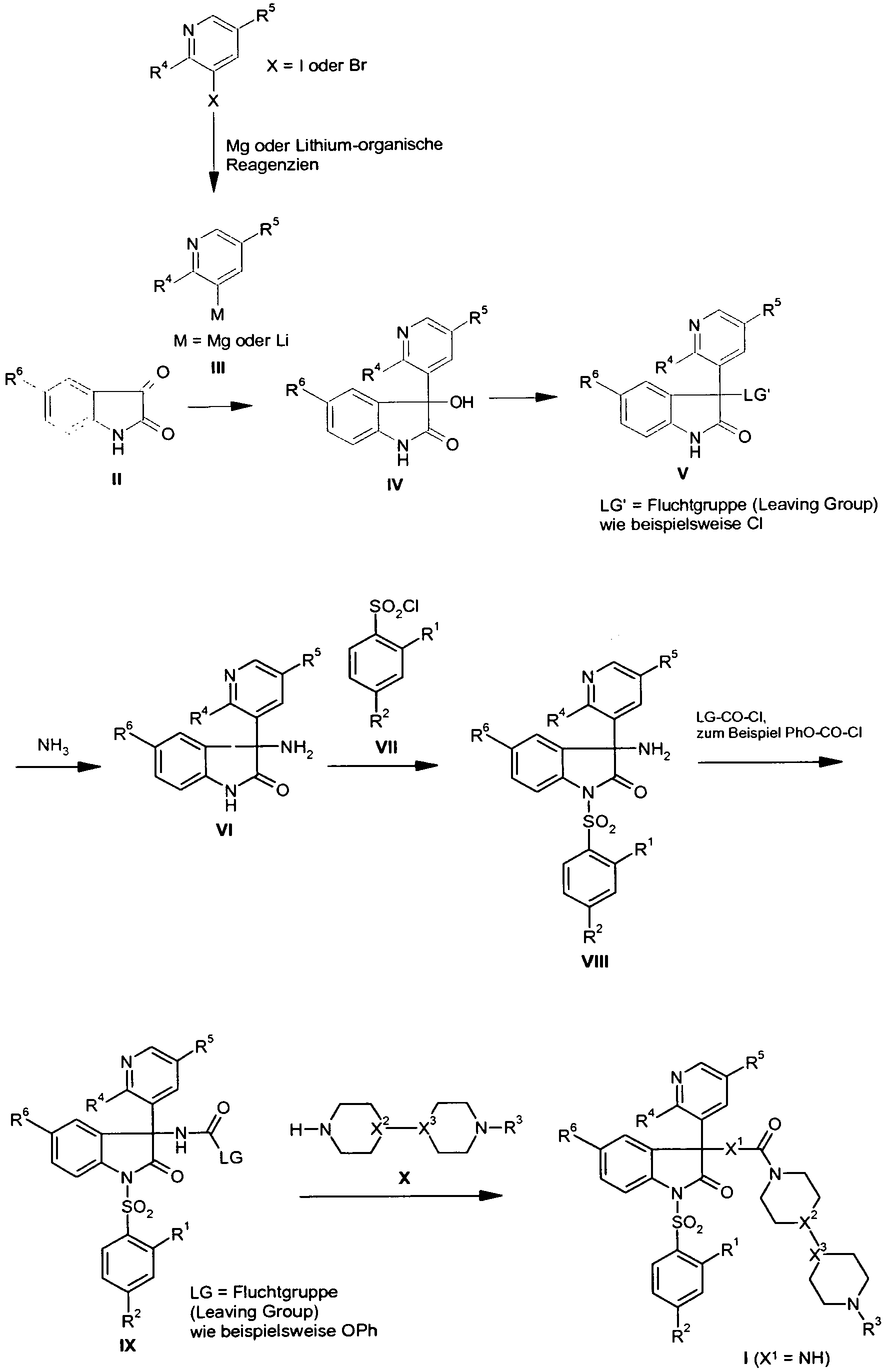

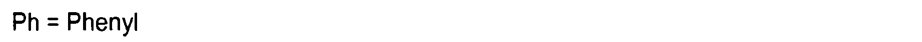

Die Herstellung der erfindungsgemäßen Verbindungen der allgemeinen Formel I, welche eine Carbamat-Gruppe in der 3-Position tragen (X¹ = O), kann wie in WO 2006/005609 beschrieben und in Syntheseschema 2 gezeigt erfolgen: Zunächst wird die 3-HydroxyVerbindung IV mit Chlorameisensäurephenylester zu den Phenylcarbonat-Derivaten Xla und / oder Xlb umgesetzt. Mit einem Überschuss an Amin X werden die Carbamat-Derivate XII erhalten, die anschliessend unter den üblichen Bedingungen (Deprotonierung mit einer starken Base, wie zum Beispiel Natriumhydrid oder Kalium-tert-butylat in einem geeigneten Lösungsmittel, wie zum Beispiel DMF, gefolgt von Behandlung mit Sulfonsäurechloriden VII) in die erfindungsgemäßen Verbindungen I mit Carbamat-Brücke überführt werden können.

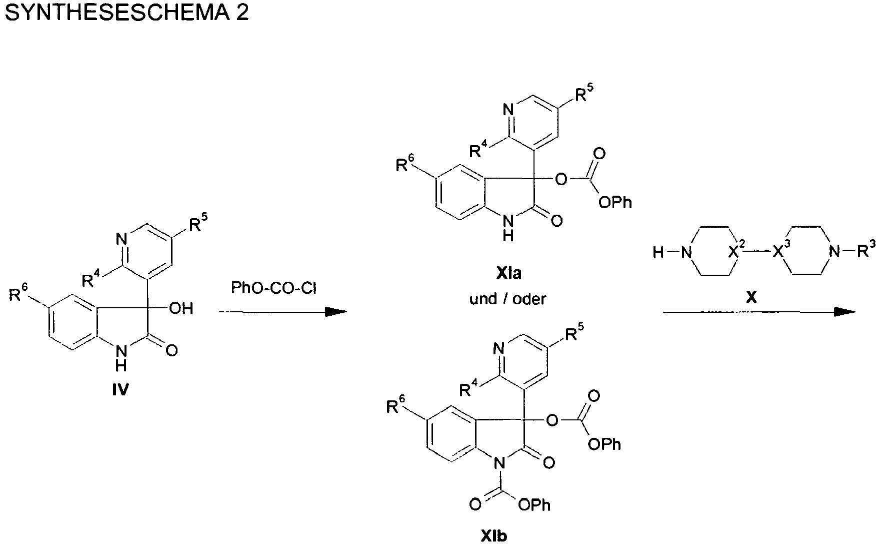

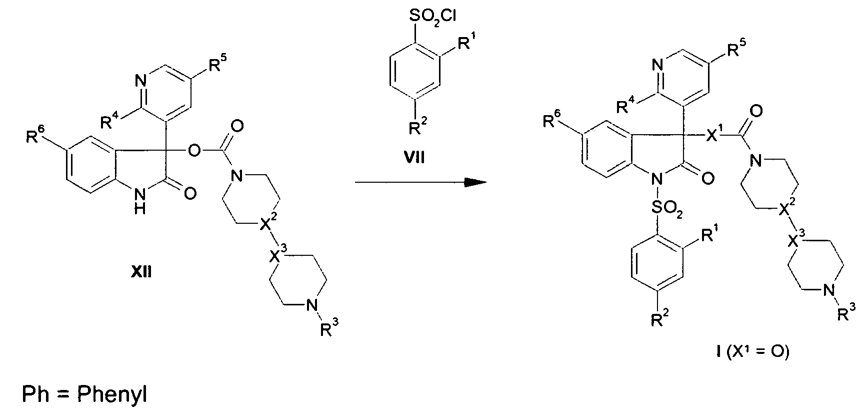

Die Herstellung der erfindungsgemäßen Verbindungen der allgemeinen Formel I, welche eine 2-Oxoethyl-Gruppe in der 3-Stellung tragen (X¹ = CH₂), kann wie in Syntheseschema 3 gezeigt erfolgen. Die Einführung der Essigsäure-Gruppierung kann wie in WO 2006/005609 beschrieben in einer 4-Stufen-Sequenz (1. Substitution der Fluchtgruppe LG' in V mit dem Natriumsalz von Dimethylmalonat, 2. Verseifung der ersten Estergruppe, 3. thermische Decarboxylierung, 4. Verseifung der zweiten Estergruppe) erfolgen. Die Amin-Seitenkette X kann unter Verwendung der aus der Peptid-Chemie bekannten Standard-Kupplungsreagenzien, wie zum Beispiel EDC (N-(3-Dimethylaminopropyl)-N'-ethylcarbodiimid-hydrochlorid) und HOBT (1-Hydroxy-benzotriazol) in einem Lösungsmittel, wie zum Beispiel N,N-Dimethylformamid, an die Carbonsäure XV gekuppelt werden. Die Sulfonylierung kann durch Deprotonierung der Kupplungsprodukte XVI mit einer starken Base, wie zum Beispiel Natriumhydrid oder Kalium-*tert*-butylat, und anschließende Behandlung mit Sulfonsäurechloriden VII in einem Lösungsmittel, wie zum Beispiel DMF, erfolgen und führt zu den erfindungsgemäßen Verbindungen I mit Amid-Brücke.

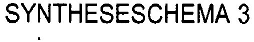

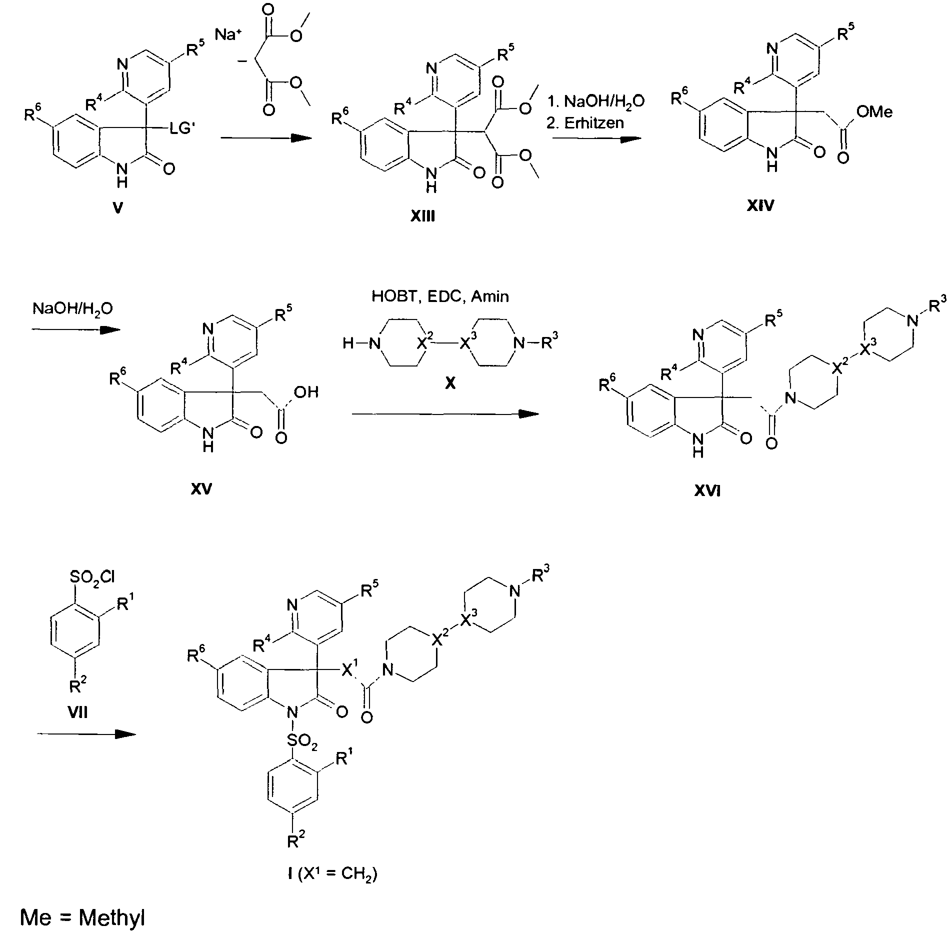

Ein weiterer Gegenstand der vorliegenden Erfindung betrifft ein pharmazeutisches Mittel, enthaltend mindestens eine Verbindung der allgemeinen Formel I und/oder ein pharmazeutisch verträgliches Salz davon, wie vorstehend ausgeführt, und einen pharmazeutisch verträglichen Träger. Geeignete Träger hängen unter anderem von der Darreichungsform des Mittels ab und sind dem Fachmann grundsätzlich bekannt. Einige geeignete Träger sind weiter unten beschrieben.

Ein weiterer Gegenstand der vorliegenden Erfindung betrifft die Verwendung von Verbindungen der Formel I und/oder von pharmazeutisch verträglichen Salzen davon zur Herstellung eines Medikaments zur Behandlung und/oder Prophylaxe von Vasopressin-abhängigen Krankheiten.

Vasopressin-abhängige Krankheiten sind solche, bei denen der Krankheitsverlauf zumindest teilweise von Vasopressin abhängt, d.h. Krankheiten, die einen erhöhten Vasopressin-Spiegel zeigen, der unmittelbar oder indirekt zum Krankheitsbild beitragen kann. Anders ausgedrückt sind Vasopressin-abhängige Krankheiten solche, die durch Modulation des Vasopressinrezeptors, beispielsweise durch Gabe eines Vasopressinrezeptorliganden (Agonist, Antagonist, partieller Antagonist/Agonist, inverser Agonist etc.), beeinflusst werden können.

In einer bevorzugten Ausführungsform betrifft die vorliegende Erfindung die Verwendung von erfindungsgemäßen Verbindungen der Formel I oder von pharmazeutisch verträglichen Salzen zur Herstellung eines Medikaments zur Behandlung und/oder Prophylaxe von Krankheiten, die ausgewählt sind unter Diabetes, Insulin-Resistenz, Enuresis nocturna, Inkontinenz und Krankheiten, bei denen Blutgerinnungsstörungen auftreten, und/oder zur Verzögerung der Miktion. Unter dem Begriff "Diabetes" sind alle Diabetesformen zu verstehen, vor allem Diabetes mellitus (einschließlich Typ I und insbesondere Typ II), Diabetes renalis und insbesondere Diabetes insipidus. Bevorzugt handelt es sich bei den Diabetesformen um Diabetes mellitus vom Typ II (mit Insulinresistenz) oder Diabetes insipidus.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung und/oder Prophylaxe von Krankheiten, die ausgewählt sind unter Hypertonie, pulmonaler Hypertonie, Herzinsuffizienz, Myocardinfarkt, koronarem Spasmus, instabiler Angina, PTCA (percutaneous transluminal coronary angioplastie), Ischämien des Herzens, Störungen des renalen Systems, Ödemen, renalem Vasospasmus, Nekrose des renalen Cortex, Hyponatriämie, Hypokaliämie, Schwartz-Bartter Syndrom, Störungen des Gastrointestinaltraktes, gastritischem Vasospasmus, Hepatozirrhose, Magen- und Darmulkus, Emesis, auftretender Emesis bei der Chemotherapie, und Reisekrankheit.

Die erfindungsgemäßen Verbindungen der Formel I bzw. ihre pharmazeutisch verträglichen Salze oder das erfindungsgemäße pharmazeutische Mittel können auch zur Behandlung von verschiedenen Vasopressin-abhängigen Beschwerden, die zentralnervöse Ursachen oder Veränderungen in der HPA-Achse (hypothalamic pituitary adrenal axis) aufweisen, verwendet werden, zum Beispiel bei affektiven Störungen, wie depressiven Störungen und bipolaren Störungen. Dazu gehören zum Beispiel dysthyme Störungen, Phobien, posttraumatische Belastungsstörungen, generelle Angsstörungen, Panikstörungen, saisonale Depressionen und Schlafstörungen.

Ebenso können die erfindungsgemäßen Verbindungen der Formel I bzw. ihre pharmazeutisch verträglichen Salze oder das erfindungsgemäße pharmazeutische Mittel zur Behandlung bei Angststörungen und stressabhängigen Angststörungen eingesetzt werden, wie zum Beispiel generalisierten Angststörungen, Phobien, posttraumatischen Angststörungen, panischen Angststörungen, obsessiv-zwanghaften Angststörungen, akuten stressabhängigen Angststörungen und Sozialphobie.

Weiterhin können die erfindungsgemäßen Verbindungen auch zur Behandlung von Gedächnisleistungsstörungen, Morbus Alzheimer, Psychosen, psychotischen Störungen, Schlafstörungen und/oder dem Cushing Syndrom sowie allen stressabhängigen Krankheiten eingesetzt werden.

Dementsprechend betrifft eine weitere bevorzugte Ausführungsform der vorliegenden Erfindung die Verwendung von erfindungsgemäßen Verbindungen der Formel I oder von pharmazeutisch verträglichen Salzen davon zur Herstellung eines Medikaments zur Behandlung von affektiven Störungen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von Angststörungen und/oder stressabhängigen Angststörungen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von Gedächtnisleistungsstörungen und/oder Morbus Alzheimer.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von Psychosen und/oder psychotischen Störungen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung des Cushing-Syndroms oder sonstigen stressabhängigen Krankheiten.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von Schlafstörungen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von depressiven Erkrankungen. Eine besondere Form von depressiven Erkrankungen sind sogenannte childhood onset mood disorders, d.h. depressive Verstimmungen, die bereits in der Kindheit einsetzen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von vasomotorischen Symptomen und/oder thermoregulatorischen Fehlfunktionen, wie beispielsweise das "hot flush"-Symptom.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung und/oder Prophylaxe von Drogen-, Arzneimittel- und/oder durch sonstige Faktoren vermittelten Abhängigkeiten, zur Behandlung und/oder Prophylaxe von Stress bedingt durch den Entzug von einem oder mehreren die Abhängigkeit vermittelnden Faktoren und/oder zur Behandlung und/oder Prophylaxe von Stress-induzierten Rückfällen in die Drogen-, Arzneimittel- und/oder durch sonstige Faktoren vermittelten Abhängigkeiten.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung und/oder Prophylaxe von Schizophrenie und/oder Psychose.

Bei dem mit dem Verfahren prophylaktisch oder therapeutisch zu behandelnden Patienten handelt es sich vorzugsweise um ein Säugetier, beispielsweise um einen Menschen oder um ein nichtmenschliches Säugetier oder um ein nichtmenschliches transgenes Säugetier. Speziell handelt es sich um einen Menschen.

Die Verbindungen der allgemeinen Formel I und ihre pharmazeutisch verträglichen Salze , wie vorstehend ausgeführt, können von einem Fachmann in Kenntnis der erfindungsgemäßen technischen Lehre in Ausführung und/oder in analoger Ausführung von an sich bekannten Verfahrensschritten hergestellt werden.

Die Verbindungen I bzw. deren pharmazeutisch verträglichen Salze zeichnen sich dadurch aus, dass sie eine Selektivität zum Vasopressin-Rezeptorsubtyp V1b gegenüber mindestens einem der nahe verwandten Vasopressin/Oxytozin-Rezeptorsubtypen (zum Beispiel Vasopressin V1a, Vasopressin V2 und/oder Oxytozin) aufweisen.

Alternativ oder vorzugsweise zusätzlich zeichnen sich die Verbindungen I bzw. deren pharmazeutisch verträglichen Salze dadurch aus, dass sie eine verbesserte metabolische Stabilität aufweisen.

Die metabolische Stabilität einer Verbindung kann beispielsweise gemessen werden, indem man eine Lösung dieser Verbindung mit Lebermikrosomen von bestimmten Spezies (zum Beispiel Ratte, Hund oder Mensch) inkubiert und die Halbwertszeit der Verbindung unter diesen Bedingungen bestimmt (RS Obach, Curr Opin Drug Discov Devel. 2001, 4, 36-44). Dabei kann aus einer beobachteten größeren Halbwertszeit auf eine verbesserte metabolische Stabilität der Verbindung geschlossen werden. Die Stabilität in Gegenwart von humanen Lebermikrosomen ist von besonderem Interesse, da sie eine Vorhersage für den metabolischen Abbau der Verbindung in der menschlichen Leber ermöglicht. Verbindungen mit erhöhter metabolischer Stabilität (gemessen in dem Lebermikrosomen-Test) werden deshalb wahrscheinlich auch in der Leber langsamer abgebaut. Der langsamere metabolische Abbau in der Leber kann zu höheren und/oder länger anhaltenden Konzentrationen (Wirkspiegel) der Verbindung im Körper führen, so dass die Eliminierungs-Halbwertszeit der erfindungsgemässen Verbindungen erhöht ist. Erhöhte und/oder länger anhaltende Wirkspiegel können zu einer besseren Wirksamkeit der Verbindung in der Behandlung oder Prophylaxe von verschiedenen Vasopressin-abhängigen Krankheiten führen. Außerdem kann eine verbesserte metabolische Stabilität zu einer erhöhten Bioverfügbarkeit nach oraler Gabe führen, da die Verbindung nach erfolgter Resorption im Darm einem geringeren metabolischen Abbau in der Leber (sogenannter "first pass effect") unterliegt. Eine erhöhte orale Bioverfügbarkeit kann aufgrund erhöhter Konzentration (Wirkspiegel) der Verbindung zu einer besseren Wirksamkeit der Verbindung nach oraler Gabe führen.

Die erfindungsgemäßen Verbindungen sind nach Verabreichung auf verschiedenen Wegen wirksam. Die Verabreichung kann beispielsweise intravenös, intramuskulär, subkutan, topisch, intratracheal, intranasal, transdermal, vaginal, rektal, sublingual, bukkal oder oral erfolgen und erfolgt häufig intravenös, intramuskulär oder insbesondere oral.

Die vorliegende Erfindung betrifft auch pharmazeutische Zusammensetzungen, die eine wirksame Dosis einer erfindungsgemäßen Verbindung I oder eines pharmazeutisch verträglichen Salzes davon und geeignete pharmazeutische Träger (Arzneiträger) enthalten.

Diese Arzneiträger werden entsprechend der pharmazeutischen Form und der gewünschten Applikationsart gewählt und sind dem Fachmann grundsätzlich bekannt.

Die erfindungsgemäßen Verbindungen der Formel I oder gegebenenfalls geeignete Salze dieser Verbindungen können zur Herstellung von pharmazeutischen Zusammensetzungen zur oralen, sublingualen, bukkalen, subkutanen, intramuskulären, intravenösen, topischen, intratrachealen, intranasalen, transdermalen, vaginalen oder rektalen Verabreichung verwendet werden und Tieren oder Menschen in einheitlichen Verabreichungsformen, gemischt mit herkömmlichen pharmazeutischen Trägern, zur Prophylaxe oder Behandlung der obigen Störungen oder Krankheiten verabreicht werden.

Die geeigneten Verabreichungsformen (Dosiereinheiten) umfassen Formen zur oralen Verabreichung, wie Tabletten, Gelatinekapseln, Pulver, Körnchen und Lösungen oder Suspensionen zur oralen Einnahme, Formen zur sublingualen, bukkalen, intratrachealen oder intranasalen Verabreichung, Aerosole, Implantate, Formen der subkutanen, intramuskulären oder intravenösen Verabreichung und Formen der rektalen Verabreichung.

Zur topischen Verabreichung können die erfindungsgemäßen Verbindungen in Cremes, Salben oder Lotionen verwendet werden.

Um den gewünschten prophylaktischen oder therapeutischen Effekt zu erzielen, kann die Dosis des Wirkstoffs zwischen 0.01 und 50 mg pro kg Körpergewicht und pro Tag variieren.

Jede Einheitsdosis kann 0.05 bis 5000 mg, vorzugsweise 1 bis 1000 mg, des Wirkstoffs in Kombination mit einem pharmazeutischen Träger enthalten. Diese Einheitsdosis kann 1-bis 5-mal am Tag verabreicht werden, so dass eine tägliche Dosis von 0,5 bis 25000 mg, vorzugsweise 1 bis 5000 mg, verabreicht wird.

Falls eine feste Zusammensetzung in Form von Tabletten zubereitet wird, wird der Wirkstoff mit einem festen pharmazeutischen Träger, wie Gelatine, Stärke, Laktose, Magnesiumstearat, Talk, Siliziumdioxid oder Ähnlichem, gemischt.

Die Tabletten können mit Saccharose, einem Cellulosederivat oder einer anderen geeigneten Substanz beschichtet werden oder anders behandelt werden, um eine anhaltende oder verzögerte Aktivität aufzuweisen und um eine vorbestimmte Menge des Wirkstoffs kontinuierlich freizugeben.

Eine Zubereitung in Form von Gelatinekapseln wird durch Mischen des Wirkstoffs mit einem Streckmittel und Aufnehmen der resultierenden Mischung in weiche oder harte Gelatinekapseln erhalten.

Eine Zubereitung in Form eines Sirups oder Elixiers oder zur Verabreichung in Form von Tropfen kann Wirkstoffe zusammen mit einem Süßstoff, der vorzugsweise kalorienfrei ist, Methylparaben oder Propylparaben als Antiseptika, einen Aromastoff und einen geeigneten Farbstoff enthalten.

Die wasserdispergierbaren Pulver oder Körnchen können die Wirkstoffe, gemischt mit Dispergiermitteln, Benetzungsmitteln oder Suspensionsmitteln, wie Polyvinylpyrrolidone, sowie Süßstoffe oder Geschmackskorrigentien, enthalten.

Eine rektale oder vaginale Verabreichung wird durch Verwendung von Zäpfchen erreicht, die mit Bindemitteln zubereitet werden, die bei rektaler Temperatur schmelzen, zum Beispiel Kakaobutter oder Polyethylenglykole. Eine parenterale Verabreichung erfolgt unter Verwendung von wässrigen Suspensionen, isotonischen Salzlösungen oder sterilen und injizierbaren Lösungen, die pharmakologisch verträgliche Dispergiermittel und/oder Benetzungsmittel, zum Beispiel Propylenglykol oder Polyethylenglykol, enthalten.

Der Wirkstoff kann auch als Mikrokapseln oder Zentrosome, falls geeignet mit einem oder mehreren Trägern oder Additiven, formuliert werden.

Zusätzlich zu den erfindungsgemäßen Verbindungen können die erfindungsgemäßen Mittel andere Wirkstoffe enthalten, die zur Behandlung der oben angegebenen Störungen oder Krankheiten nützlich sein können.

Die vorliegende Erfindung betrifft somit weiterhin pharmazeutische Mittel, in denen mehrere Wirkstoffe zusammen anwesend sind, wobei mindestens einer von diesen eine erfindungsgemäße Verbindung I oder Salz davon ist.

Die Erfindung wird nachstehend anhand von Beispielen näher erläutert, wobei die Beispiele nicht einschränkend zu verstehen sind.

Die Herstellung der erfindungsgemäßen Verbindungen kann über verschiedene Syntheserouten erfolgen. Die genannten Vorschriften, wie entsprechend in den Syntheseschemata 1, 2 und 3 beschrieben, sind nur exemplarisch anhand der genannten Beispiele näher erläutert, ohne ausschließlich auf die genannten Synthesewege 1, 2 oder 3 oder Analogvorschriften beschränkt zu sein.

### EXPERIMENTELLER TEIL

### Verwendete Abkürzungen:

- THF:: Tetrahydrofuran
- DMSO:: Dimethylsulfoxid
- TFA:: Trifluoressigsäure
- p:: pseudo (beispielsweise pt pseudo Triplett)
- b:: breit (beispielsweise bs breites Singulett)
- s:: Singulett
- d:: Doublett
- t:: Triplett
- m:: Multiplett
- dd:: doppeltes Doublett
- dt:: doppeltes Triplett
- tt:: dreifaches Triplett

### I. Herstellung der Ausgangsverbindungen

### a) 3-Hydroxvindole der allgemeinen Formel IV

### a.1 5-Chlor-3-(2-ethoxy-pyridin-3-y)-3-hydroxy-1,3-dihydroindol-2-on

Bildung des Isatin-Natriumsalzes: Zu 13.9 g (76.5 mmol) 5-Chlorisatin in 300 ml THF gab man bei 0 °C portionsweise 3.21 g (80.3 mmol) Natriumhydrid (60% in Mineralöl) und rührte eine Stunde bei 0 °C.

Bildung des Grignard-Reagenzes: Zu einer Lösung von 2-Ethoxy-3-iodpyridin (20.0 g, 80.3 mmol) in 250 ml THF tropfte man Ethylmagnesiumbromid (95.6 mmol, 95.6 ml einer 1 M Lösung in THF), wobei man die Temperatur zwischen 15 und 22 °C hielt. Anschließend rührte man das Reaktionsgemisch 15 min bei Raumtemperatur.

Grignard-Addition: Man pumpte die Lösung des Grignard-Reagenzes zu der eisgekühlten Lösung des Isatin-Natriumsalzes und rührte das Reaktionsgemisch anschließend drei Stunden bei Raumtemperatur. Man goss den Ansatz in 10% Ammoniumchlorid-Lösung und extrahierte dreimal mit Essigester. Die vereinigten organischen Phasen wurden mit Wasser und gesättigter Kochsalzlösung gewaschen, mit Magnesiumsulfat getrocknet und unter vermindertem Druck eingeengt. Der nach Stehen über Nacht bei Raumtemperatur gebildete kristalline Niederschlag wurde abgesaugt und mit Essigester gewaschen. Man erhielt 13.3 g der Titelverbindung als rötlichen Feststoff.
ESI-MS [M+H⁺] = 305 (CI-Isotopenmuster).

### a.2 5-Chlor-3-(2-ethoxy-5-methyl-pyridin-3-yl)-3-hydroxy-1,3-dihydroindol-2-on

Bildung des Isatin-Natriumsalzes: Zu 5.0 g (27.5 mmol) 5-Chlorisatin in 100 ml THF gab man bei 0 °C portionsweise 1.01 g (27.5 mmol) Natriumhydrid (60% in Mineralöl) und rührte eine Stunde bei 0 °C.

Bildung des Lithium-Reagenzes: Zu einer auf -78 °C gekühlten Lösung von 3-Brom-2-ethoxy-5-methylpyridin (7.74 g, 35.8 mmol) in 100 ml THF tropfte man n-Butyllithium (37.2 mmol, 23.2 ml einer 1.6 M Lösung in Hexan), wobei man die Temperatur unter-60 °C hielt und rührte danach das Reaktionsgemisch weitere 15 min im Aceton / Trockeneis-Bad.

Addition: Die auf -78 °C gekühlte Lösung des lithiierten Pyridins wurde mittels einer Transfernadel zu der eisgekühlten Lösung des Isatin-Natriumsalzes gepumpt und das Reaktionsgemisch anschließend drei Stunden bei Raumtemperatur gerührt. Man goss die Reaktionsmischung in 10% Ammoniumchlorid-Lösung und extrahierte dreimal mit Essigester. Die vereinigten organischen Phasen wusch man mit Wasser und gesättigter Kochsalzlösung, trocknete über Magnesiumsulfat und engte unter vermindertem Druck ein. Nach Behandlung des Rückstandes mit Dichlormethan bildete sich ein beiger Niederschlag, den man absaugte und mit Dichlormethan wusch. Man erhielt: 2.61 g der Titelverbindung als rötlichen Feststoff.
ESI-MS [M+H⁺] = 319 (CI-Isotopenmuster).

### a.3 5-Chlor-3-(2-isopropoxy-pyridin-3-yl)-3-hydroxy-1,3-dihydroindol-2-on

Man stellte die Titelverbindung analog zu dem Verfahren aus Beispiel a.1 unter Verwendung von 3-lod-2-isopropoxypyridin her.

### b) Amine der allgemeinen Formel X

### b.1 1-Ethyl-4-piperidin-4-yl-piperazin

### b.1.1 tert-Butyl 4-(4-ethylpiperazin-1-yl)piperidin-1-carboxylat

29.2 g (256 mmol) N-Ethylpiperazin wurden mit 50.0 g (256 mmol) tert-Butyl 4-oxopiperidin-1-carboxylat (entspricht 1-Boc-4-piperidon) unter Eiskühlung in 800 ml Ethanol vorgelegt. Man gab 15.4 g (256 mmol) Eisessig zu. Anschließend wurden portionsweise 16.1 g (256 mmol) Natriumacetoxyborhydrid zur gekühlten Reaktionsmischung gegeben. Zunächst war eine leichte Gasbildung und nach 2/3-Zugabe des Reduktionsmittels eine Schaumbildung zu beobachten. Die Reaktionsmischung wurde über Nacht bei Raumtemperatur gerührt. Zur Aufarbeitung wurde die Reaktionslösung unter Kühlung mit 200 ml 2 N Natronlauge versetzt, das Lösungsmittel Ethanol abdestilliert und die übrigbleibende Reaktionsmischung mit Wasser verdünnt. Es wurde mit Diethylether (2 x) extrahiert, mit gesättigter Natriumchlorid-Lösung (1 x) gewaschen, und die vereinigten organischen Phasen über Magnesiumsulfat getrocknet, filtriert und das Lösungsmittel im Vakuum entfernt. Die rohe Titelverbindung wurde als gelbes Öl erhalten, das anschließend über eine mit Kieselgel gefüllte, 4 I-Nutsche mit Dichlormethan und 10 % Methanol als Elutionsmittel chromatographiert wurde. Es wurden insgesamt 40 g (135 mmol, 53 %) tert-Butyl 4-(4-ethylpiperazin-1-yl)piperidin-1-carboxylat erhalten.

### b.1.2 1-Ethyl-4-piperidin-4-yl-piperazin als Chlorid-Salz

Zur Schutzgruppenentfemung wurden 40 g (135 mmol) tert-Butyl 4-(4-ethylpiperazin-1-yl)piperidin-1-carboxylat aus Beispiel b.1.1 in 200 ml Methanol und 1.8 I Dichlormethan vorgelegt und mit 100 ml 5-6 *M* HCl-Lösung in Isopropanol versetzt. Es entstand eine Suspension, wobei auch eine leichte Gasentwicklung zu beobachten war. Der Reaktionsansatz wurde eine Stunde bei 40 °C (Wasserbadtemperatur) gerührt und 48 Stunden bei Raumtemperatur nachgerührt. Zur vollständigen Entschützung wurden nochmals 50 ml der 5-6 M HCl-Lösung in Isopropanol zugegeben und die Reaktionsmischung bei 40°C gerührt. Am Rotationsverdampfer wurde das Dichlormethan abdestilliert. Es wurden nochmals 200 ml Methanol und 30 ml der 5-6 M HCl-Lösung in Isopropanol zugesetzt. Man rührte die Reaktionsmischung eine Stunde am Rückfluss, wobei sich eine weiße Suspension mit starker Gasentwicklung bildete. Danach entstand eine dünnflüssige Suspension, die auf Raumtemperatur abgekühlt wurde. Der Niederschlag wurde abgesaugt und mit Methanol und Diethylether gewaschen. Nach dem Trocknen wurden 36 g (117 mmol, 87 %) des 1-Ethyl-4-piperidin-4-yl-piperazins als Chlorid-Salz isoliert.
¹H-NMR (D₂O, 400 MHz) δ [ppm] = 3.74 - 3.47 (m, 11 H), 3.28 (q, 2H, J = 7.3 Hz), 3.06 (dt, 2H, J = 2.2 Hz, J = 13.2 Hz), 2.38 (m, 2H, J = 13.6 Hz), 1.89 (dq, 2H, J = 4.1 Hz, J = 13.3 Hz), 1.30 (t, 3H, J = 7.3 Hz).

### II. Herstellung der racemischen Verbindungen der Formel I

Die erfindungsgemäßen Verbindungen I wurden in einigen Fällen durch präparative HPLC gereinigt, zum Beispiel über eine Prontosil Prep 2012, C18, 125x20 mm, 5µm Säule mit einem Laufmittel-Gradienten von 10% bis 100% Acetonitril in Wasser und 0.1% Trifluoressigsäure als Modulator. Die Verbindungen I fallen dann als Trifluoressigsäure-Salz an.

### II.1 Herstellung der racemischen Verbindungen der Formel I, worin X¹ für NH steht (Beispiele 1 bis 21)

### BEISPIEL 1:

### (±)-4-(1-Methyl-piperidin-4-yl)-piperazin-1-carbonsäure [5-chlor-1-(2,4-dimethoxyphenylsulfonyl)-3-(2-ethoxy-pyridin-3-yl)-2-oxo-2,3-dihydro-1H-indol-3-yl]-amid

### 1.1 3,5-Dichlor-3-(2-ethoxy-pyridin-3-yl)-1,3-dihydroindol-2-on

Zu einer Suspension von 13.3 g (43.6 mmol) 5-Chlor-3-(2-ethoxy-pyridin-3-yl)-3-hydroxy-1,3-dihydroindol-2-on aus Beispiel a.1 in 50 ml Dichlormethan gab man 4.4 ml (54.3 mmol) Pyridin. Nach dem Kühlen der Reaktionsmischung auf 0 °C tropfte man 3.8 ml (52.3 mmol) Thionylchlorid zu. Man rührte die Mischung bei Raumtemperatur eine Stunde und goss anschließend in Eiswasser. Nach 15 Minuten Rühren trennte man die organische Phase ab. Die wässrige Phase wurde mehrmals mit Dichlormethan extrahiert. Man trocknete die vereinigte organische Phase über Magnesiumsulfat, filtrierte und entfernte das Lösungsmittel im Vakuum. Man erhielt 15.6 g der Titelverbindung als gelblichen Feststoff, der ohne weitere Aufreinigung in der nächsten Stufe eingesetzt wurde.

### 1.2 3-Amino-5-chlor-3-(2-ethoxy-pyridin-3-yl)-1,3-dihydroindol-2-on

Zu einer gekühlten Lösung von 47 g (145 mmol) 3,5-Dichlor-3-(2-ethoxy-pyridin-3-yl)-1,3-dihydroindol-2-on in 250 ml Dichlormethan tropfte man unter Stickstoffatmosphäre 104 ml (728 mmol) einer 7 N methanolischen Ammoniaklösung und rührte danach das Reaktionsgemisch über Nacht bei Raumtemperatur. Man versetzte das Reaktionsgemisch mit 250 ml Wasser und 250 ml Dichlormethan. Nach 5 min. Rühren fiel ein weißer Feststoff aus, der abfiltriert und mit Wasser und Dichlormethan gewaschen wurde. Nach Trocknen im Vakuumtrockenschrank erhielt man 26 g der Titelverbindung als weißen Feststoff.
ESI-MS [M+H⁺] = 304 (CI-Isotopenmuster)

### 1.3 3-Amino-5-chlor-1-(2,4-dimethoxy-phenylsulfonyl)-3-(2-ethoxy-pyridin-3-yl)-1,3-dihydroindol-2-on

Zu einer Lösung von 20 g (66 mmol) 3-Amino-5-chlor-3-(2-ethoxy-pyridin-3-yl)-1,3-dihydroindol-2-on in 50 ml wasserfreiem Dimethylformamid gab man unter Stickstoffatmosphäre und unter Kühlung durch ein Eisbad 3.2 g (79 mmol) Natriumhydrid (60% Dispersion in Mineralöl) portionsweise zu. Man rührte 10 min bei 0 °C und gab danach 18.7 g (79 mmol) 2,4-Dimethoxy-benzolsulfonylchlorid zu und rührte 30 min. bei Raumtemperatur nach. Die Reaktionsmischung wurde in Eiswasser gegossen und anschließend mit Essigester extrahiert. Man wusch die organische Phase mit gesättigter Natriumchlorid-Lösung, trocknete über Magnesiumsulfat und verdampfte das Lösungsmittel. Der Rückstand wurde chromatographisch über Kieselgel (120 g Redisep-Kartusche, Laufmittel-Gradient von 2 bis 25% Dichlormethan in Essigester) gereinigt. Man erhielt 34 g der Titelverbindung als weißen Feststoff.
ESI-MS [M+H⁺] = 504 (Cl-Isotopenmuster)

### 1.4 [5-Chlor-1-(2,4-dimethoxy-phenylsulfonyl)-3-(2-ethoxy-pyridin-3-yl)-2-oxo-2,3-dihydro-1H-indol-3-yl]-carbaminsäurephenylester

Zu einer auf 0 °C gekühlten Lösung von 2.57 g (5.1 mmol) 3-Amino-5-chlor-1-(2,4-dimethoxy-phenylsulfonyl)-3-(2-ethoxy-pyridin-3-yl)-1,3-dihydroindol-2-on in 10 ml Dichlormethan und 4.1 ml Pyridin tropfte man langsam 0.83 ml (6.6 mmol) Chlorameisensäurephenylester. Danach rührte man die Reaktionsmischung über Nacht bei Raumtemperatur. Man verdünnte das Reaktionsgemisch mit Dichlormethan und extrahierte mit Wasser. Die organische Phase wurde mit Wasser und gesättigter Kochsalzlösung gewaschen, über Magnesiumsulfat getrocknet und unter vermindertem Druck eingeengt. Man löste den Rückstand in wenig Dichlormethan und gab das sechsfache Volumen an Diisopropylether zu. Man filtrierte den entstehenden Niederschlag ab, wusch mit Diisopropylether und trocknete im Vakuum. Man erhielt 3.01 g der Titelverbindung als weißen Feststoff.
ESI-MS [M+H⁺] = 624 (Cl-Isotopenmuster).

### 1.5 4-(1-Methy)-piperidin-4-yl)-piperazin-1-carbonsäure [5-chlor-1-(2,4-dimethoxyphenylsulfonyl)-3-(2-ethoxy-pyridin-3-yl)-2-oxo-2,3-dihydro-1H-indol-3-yl]-amid

Man rührte eine Mischung aus 800 mg (1.28 mmol) [5-Chlor-1-(2,4-dimethoxy-phenylsulfonyl)-3-(2-ethoxy-pyridin-3-yl)-2-oxo-2,3-dihydro-1H-indol-3-yl]-carbaminsäurephenylester, 470 mg (2.56 mmol) 1-(1-Methylpiperidin-4-yl)-piperazin und 5 ml getrocknetem THF 24 Stunden bei Raumtemperatur. Man verdünnte das Reaktionsgemisch mit Dichlormethan, wusch mit Wasser und gesättigter Kochsalzlösung, trocknete die organische Phase über Magnesiumsulfat und engte unter vermindertem Druck ein. Der Rückstand wurde chromatographisch über Kieselgel (12 g Redisep-Kartusche, Laufmittel-Gradient von 2 bis 12% Methanol in Dichlormethan) gereinigt. Man erhielt 790 mg der Titelverbindung als weißen Feststoff.
ESI-MS [M+H⁺] = 713 (CI-Isotopenmuster)
¹H-NMR ([d₆]-DMSO, 500 MHz) δ [ppm] = 8.10 (1H), 7.85 (1H), 7.70 (1H), 7.55 (2H), 7.35 (2H), 6.95 (1H), 6.65 (2H), 4.20 (2H), 3.85 (3H), 3.45 (3H), 3.20 (4H), 2.75 (2H), 2.35 (4H), 2.10 (4H), 1.80 (2H), 1.65 (2H), 1.35 (2H), 1.15 (3H).

Die Verbindungen der Formel I, worin X¹ für NH steht, gemäß den Beispielen 2 bis 21 können unter Verwendung der entsprechenden 3-Hydroxyoxindole der Formel IV, Sulfonylchloride der Formel VII sowie Amine der Formel X analog zum Herstellungsverfahren des Beispiels 1 hergestellt werden.

### BEISPIEL 2:

### (±)-4-(1-Methyl-piperidin-4-yl)-piperazin-1-carbonsäure [5-chlor-3-(2-ethoxy-pyridin-3-yl)-1-(2-methoxy-phenylsulfonyl)-2-oxo-2,3-dihydro-1H-indol-3-yl]-amid, Trifluoressigsäure-Salz

ESI-MS [M+H]⁺ = 683 (Cl-Isotopenmuster)

### BEISPIEL 3:

### (±)-4-(1-Methyl-piperidin-4-yl)-piperazin-1-carbonsäure [1-phenylsulfonyl-5-chlor-3-(2-ethoxy-pyridin-3-yl)-2-oxo-2,3-dihydro-1H-indol-3-yl]-amid, Trifluoressigsäure-Salz A-977409

ESI-MS [M+H⁺] = 653 (Cl-Isotopenmuster)

### BEISPIEL 4:

### (±)-4-(1-Ethyl-piperidin-4-yl)-piperazin-1-carbonsäure [5-chlor-1-(2,4-dimethoxyphenylsulfonyl)-3-(2-ethoxy-pyridin-3-yl)-2-oxo-2,3-dihydro-1H-indol-3-yl]-amid ESI-MS [M+H⁺] = 727 (Cl-Isotopenmuster)

¹H-NMR ([d₆]-DMSO, 500 MHz) δ [ppm] = 8.10 (1H), 7.85 (1H), 7.70 (1H), 7.55 (2H), 7.35 (2H), 6.95 (1H), 6.65 (2H), 4.20 (2H), 3.85 (3H), 3.45 (3H), 3.20 (4H), 2.85 (2H), 2.35 (4H), 2.30 (2H), 2.10 (1H), 1.80 (2H), 1.65 (2H), 1.35 (2H), 1.10 (3H), 0.95 (3H).

### BEISPIEL 5:

### (±)-4-(4-Methyl-piperazin-1-yl)-piperidin-1-carbonsäure [5-chlor-1-(2,4-dimethoxyphenylsulfonyl)-3-(2-ethoxy-pyridin-3-yl)-2-oxo-2,3-dihydro-1H-indol-3-yl]-amid ESI-MS [M+H⁺] = 713 (CI-Isotopenmuster)

¹H-NMR ([d₆]-DMSO, 500 MHz) δ [ppm] = 8.10 (1H), 7.90 (1H), 7.70 (1H), 7.60 (1H), 7.55 (1H), 7.35 (2H), 6.95 (1H), 6.65 (2H), 4.25 (2H), 3.85 (3H), 3.80 (2H), 3.45 (3H), 2.65 (2H), 2.40 (4H), 2.30 (4H), 2.15 (3H), 1.60 (2H), 1.15 (5H).

### BEISPIEL 6:

### (±)-4-(4-Methyl-piperazin-1-yl)-piperidin-1-carbonsäure[5-chlor-3-(2-ethoxy-pyridin-3-yl)-1-(2-methoxy-phenylsulfonyl)-2-oxo-2,3-dihydro-1H-indol-3-yl]-amid, Trifluoressigsäure-Salz

ESI-MS [M+H⁺] = 683 (CI-Isotopenmuster)

### BEISPIEL 7:

### (±)-4-(4-Methyl-piperazin-1-yl)-piperidin-1-carbonsäure[1-phenylsulfonyl-5-chlor-3-(2-ethoxy-pyridin-3-yl)-2-oxo-2,3-dihydro-1 H-indol-3-yl]-amid, Trifluoressigsäure-Salz

ESI-MS [M+H⁺] = 653 (Cl-Isotopenmuster)

### BEISPIEL 8:

### (±)-4-(4-Ethyl-piperazin-1-yl)-piperidin-1-carbonsäure [5-chlor-1-(2,4-dimethoxyphenylsulfonyl)-3-(2-ethoxy-pyridin-3-yl)-2-oxo-2,3-dihydro-1H-indol-3-yl]-amid

ESI-MS [M+H⁺] = 727 (CI-Isotopenmuster)
¹H-NMR ([d₆]-DMSO, 500 MHz) δ [ppm] = 8.10 (1H), 7.90 (1H), 7.70 (1H), 7.60 (1H), 7.55 (1H), 7.35 (2H), 6.95 (1H), 6.65 (2H), 4.20 (2H), 3.85 (3H), 3.80 (2H), 3.45 (3H), 2.65 (2H), 2.25-2.50 (11H), 1.65 (2H), 1.15 (5H), 0.95 (3H).

### BEISPIEL 9:

### (±)-4-(4-Ethyl-piperazin-1-yl)-piperidin-1-carbonsäure [5-chlor-3-(2-ethoxy-pyridin-3-yl)-1-(2-methoxy-phenylsulfonyl)-2-oxo-2,3-dihydro-1H-indol-3-yl]-amid, Trifluoressigsäure-Salz

ESI-MS [M+H⁺] = 697 (Cl-Isotopenmuster)

### BEISPIEL 10:

### (±)-4-(4-Ethyl-piperazin-1-yl)-piperidin-1-carbonsäure [1-phenylsulfonyl-5-chlor-3-(2-ethoxy-pyridin-3-yl)-2-oxo-2,3-dihydro-1H-indol-3-yl]-amid, Trifluoressigsäure-Salz

ESI-MS [M+H⁺] = 667 (Cl-Isotopenmuster)

### BEISPIEL 11:

### (±)-[4,4']Bipiperidinyl-1-carbonsäure [5-chlor-1-(2,4-dimethoxy-phenylsulfonyl)-3-(2-ethoxy-pyridin-3-yl)-2-oxo-2,3-dihydro-1H-indol-3-yl]-amid, Trifluoressigsäure-Salz

ESI-MS [M+H⁺] = 698 (Cl-Isotopenmuster)

### BEISPIEL 12:

### (±)-1'-Ethyl-[4,4']bipiperidinyl-1-carbonsäure [5-chlor-1-(2,4-dimethoxy-phenylsulfonyl)-3-(2-ethoxy-pyridin-3-yl)-2-oxo-2,3-dihydro-1H-indol-3-yl]-amid

ESI-MS [M+H⁺] = 726 (Cl-Isotopenmuster)

### BEISPIEL 13:

### (±)-1'-Methyl-[4,4']bipiperidinyl-1-carbonsäure[5-chlor-1-(2,4-dimethoxy-phenylsulfonyl)-3-(2-ethoxy-pyridin-3-yl)-2-oxo-2,3-dihydro-1H-indol-3-yl]-amid

ESI-MS [M+H⁺] = 712 (Cl-Isotopenmuster)
¹H-NMR ([d₆]-DMSO, 500 MHz) δ [ppm] = 8.10 (1H), 7.90 (1H), 7.70 (1H), 7.60 (1H), 7.55 (1H), 7.40 (1H), 7.35 (1H), 7.00 (1H), 6.65 (2H), 4.20 (2H), 3.90 (2H), 3.85 (3H), 3.45 (3H), 2.90 (2H), 2.55 (2H), 2.25 (3H), 2.05 (2H), 1.55 (4H), 1.25 (3H), 1.15 (3H), 0.95 (3H).

### BEISPIEL 14:

### (±)-4-(1-Methyl-piperidin-4-yl)-piperazin-1-carbonsäure [5-chlor-1-(2,4-dimethoxyphenylsulfonyl)-3-(2-ethoxy-5-methyl-pyridin-3-yl)-2-oxo-2,3-dihydro-1H-indol-3-yl]-amid, Trifluoressigsäure-Salz

ESI-MS [M+H⁺] = 727 (Cl-Isotopenmuster)
¹H-NMR ([d₆]-DMSO, 500 MHz) δ [ppm] = 9.64 (1H), 7.98 (1H), 7.93 (1H), 7.89 (1H), 7.74 (1H), 7.40 (2H), 7.32 (1H), 6.75 (1H), 6.70 (1H), 4.20 (2H), 3.88 (3H), 3.60 (2H), 3.48 (3H), 2.85-3.40 (9H), 2.80 (3H), 2.30 (2H), 2.17 (3H), 1.80 (2H), 1.15 (3H).

### BEISPIEL 15:

### (±)-4-(1-Ethyl-piperidin-4-yl)-piperazin-1-carbonsäure[5-chlor-1-(2,4-dimethoxyphenylsulfonyl)-3-(2-ethoxy-5-methyl-pyridin-3-yl)-2-oxo-2,3-dihydro-1H-indol-3-yl]-amid, Trifluoressigsäure-Salz

ESI-MS [M+H⁺] = 741 (Cl-Isotopenmuster)

### BEISPIEL 16:

### (±)-4-(4-Methyl-piperazin-1-yl)-piperidin-1-carbonsäure [5-chlor-1-(2,4-dimethoxyphenylsulfonyl)-3-(2-ethoxy-5-methyl-pyridin-3-yl)-2-oxo-2,3-dihydro-1H-indol-3-yl]-amid, Trifluoressigsäure-Salz

ESI-MS [M+H⁺] = 727 (Cl-Isotopenmuster)

### BEISPIEL 17:

### (±)-4-(4-Ethyl-piperazin-1-yl)-piperidin-1-carbonsäure[5-chlor-1-(2,4-dimethoxyphenylsulfonyl)-3-(2-ethoxy-5-methyl-pyridin-3-yl)-2-oxo-2,3-dihydro-1H-indol-3-yl]-amid, Trifluoressigsäure-Salz

ESI-MS [M+H⁺] = 741 (CI-Isotopenmuster)

### BEISPIEL 18:

### (±)-4-(1-Methyl-piperidin-4-yl)-piperazin-1-carbonsäure [5-chlor-1-(2,4-dimethoxyphenylsulfonyl)-3-(2-isopropoxy-pyridin-3-yl)-2-oxo-2,3-dihydro-1H-indol-3-yl]-amid ESI-MS [M+H⁺] = 727 (Cl-Isotopenmuster)

¹H-NMR ([d₆]-DMSO, 500 MHz) δ [ppm] = 8.10 (1H), 7.85 (1H), 7.70 (1H), 7.60 (1H), 7.50 (1H), 7.40 (1H), 7.35 (1H), 6.95 (1H), 6.70 (1H), 6.65 (1H), 5.20 (1H), 3.85 (3H), 3.45 (3H), 3.20 (4H), 2.75 (2H), 2.35 (4H), 2.10 (4H), 1.80 (2H), 1.65 (2H), 1.35 (2H), 1.20 (3H), 1.05 (3H).

### BEISPIEL 19:

### (±)-4-(1-Ethyl-piperidin-4-yl)-piperazin-1-carbonsäure [5-chlor-1-(2,4-dimethoxyphenylsulfonyl)-3-(2-isopropoxy-pyridin-3-yl)-2-oxo-2,3-dihydro-1H-indol-3-yl]-amid

ESI-MS [M+H⁺] = 741 (Cl-Isotopenmuster)

### BEISPIEL 20:

### (±)-4-(4-Methyl-piperazin-1-yl)-piperidin-1-carbonsäure [5-chlor-1-(2,4-dimethoxyphenylsulfonyl)-3-(2-isopropoxy-pyridin-3-yl)-2-oxo-2,3-dihydro-1H-indol-3-yl]-amid

ESI-MS [M+H⁺] = 727 (Cl-Isotopenmuster)

### BEISPIEL 21:

### (±)-4-(4-Ethyl-piperazin-1-yl)-piperidin-1-carbonsäure [5-chlor-1-(2,4-dimethoxyphenylsulfonyl)-3-(2-isopropoxy-pyridin-3-yl)-2-oxo-2,3-dihydro-1H-indol-3-yl]-amid

ESI-MS [M+H⁺] = 741 (Cl-Isotopenmuster)

### 11.2 Herstellung der racemischen Verbindungen der Formel I, worin X¹ für O steht (Beispiele 22 bis 27)

### BEISPIEL 22:

### (±)-4-(1-Methyl-piperidin-4-yl)-piperazin-1-carbonsäure 5-chlor-1-(2,4-dimethoxyphenylsulfonyl)-3-(2-ethoxy-pyridin-3-yl)-2-oxo-2,3-dihydro-1H-indol-3-yl-ester, Trifluoressigsäure-Salz

### 22.1 5-Chlor-3-(2-ethoxy-pyridin-3-yl)-2-oxo-3-phenoxycarbonyloxy-2,3-dihydroindol-1-carbonsäurephenylester

Zu einer auf 0 °C gekühlten Lösung von 3.00 g (9.8 mmol) 5-Chlor-3-(2-ethoxypyridin-3-yl)-3-hydroxy-1,3-dihydroindol-2-on aus Beispiel a.1 in 10 ml Pyridin tropfte man langsam 2.6 ml (21 mmol) Chlorameisensäurephenylester und rührte die Reaktionsmischung über Nacht bei Raumtemperatur nach. Man verdünnte das Reaktionsgemisch mit Dichlormethan und extrahierte mit Wasser. Man wusch die organische Phase mit Wasser und gesättigter Kochsalzlösung, trocknete über Magnesiumsulfat und engte unter vermindertem Druck ein. Der Rückstand wurde mit Diethylether ausgerührt. Man erhielt 2.58 g der Titelverbindung als weißen Feststoff.
ESI-MS [M+H⁺] = 545 (CI-Isotopenmuster).

### 22.2 4-(1-Methyl-piperidin-4-yl)-piperazin-1-carbonsäure 5-chlor-3-(2-ethoxy-pyridin-3-yl)-2-oxo-2,3-dihydro-1H-indol-3-yl ester

Eine Mischung aus 1.29 g (2.37 mmol) 5-Chlor-3-(2-ethoxy-pyridin-3-yl)-2-oxo-3-phenoxycarbonyloxy-2,3-dihydroindol-1-carbonsäurephenylester, 1.73 g (9.46 mmol) 1-(1-Methylpiperidin-4-yl)-piperazin und 10 ml getrocknetes THF wurde 72 Stunden bei Raumtemperatur gerührt. Man verdünnte das Reaktionsgemisch mit Dichlormethan und extrahierte mit Wasser und gesättigter Kochsalzlösung. Man trocknete die organische Phase über Magnesiumsulfat und engte unter vermindertem Druck ein. Den Rückstand rührte man mit Diethylether aus. Man erhielt 963 mg der Titelverbindung als weißen Feststoff.
ESI-MS [M+H⁺] = 514 (Cl-Isotopenmuster).

### 22.3 4-(1-Methyl-piperidin-4-yl)-piperazin-l-carbonsäure 5-chlor-1-(2,4-dimethoxyphenylsulfonyl)-3-(2-ethoxy-pyridin-3-yl)-2-oxo-2,3-dihydro-1H-indol-3-yl ester, Trifluoressigsäure-Salz

Zu einer Lösung von 80 mg (0.16 mmol) 4-(1-Methyl-piperidin-4-yl)-piperazin-1-carbonsäure 5-chlor-3-(2-ethoxy-pyridin-3-yl)-2-oxo-2,3-dihydro-1H-indol-3-yl ester in 2 ml wasserfreiem Dimethylformamid gab man unter Stickstoffatmosphäre und unter Kühlung durch ein Eisbad 7.5 mg (0.19 mmol) Natriumhydrid (60% Dispersion in Mineralöl). Man rührte 10 min. bei 0 °C, gab anschließend 44 mg (0.19 mmol) 2,4-Dimethoxybenzolsulfonylchlorid zu und rührte weitere 30 min bei Raumtemperatur. Man goss die Reaktionsmischung in Eiswasser und extrahierte anschließend mit Essigester. Die organische Phase wusch man mit gesättigter Natriumchlorid-Lösung, trocknete über Magnesiumsulfat und verdampfte das Lösungsmittel. Der Rückstand wurde durch präparative HPLC (Prontosil Prep 2012, C18, 125x20 mm, 5µm, Gradient von 10% bis 100% Acetonitril in Wasser, 0.1 % Trifluoressigsäure) gereinigt. Man erhielt 78 mg der Titelverbindung als weißen Feststoff.
ESI-MS [M+H⁺] = 714 (CI-Isotopenmuster)
¹H-NMR ([d₆]-DMSO, 500 MHz) δ [ppm] = 9.60 (1H), 8.20 (1H), 8.15 (1H), 7.85 (1H), 7.80 (1H), 7.50 (1H), 7.20 (1H), 7.10 (1H), 6.70 (2H), 4.10 (2H), 3.85 (3H), 3.55 (3H), 2.90-3.50 (11H), 2.75 (3H), 2.15 (2H), 1.75 (2H), 1.00 (3H).

Die Verbindungen der Formel I, worin X¹ für O steht, gemäß den Beispielen 23 bis 27 können unter Verwendung der entsprechenden 3-Hydroxyoxindole der Formel IV, Sulfonylchloride der Formel VII sowie Amine der Formel X analog zum Herstellungsverfahren des Beispiels 22 hergestellt werden.

### BEISPIEL 23:

### (±)-4-(1-Methyl-piperidin-4-yl)-piperazin-1-carbonsäure5-chlor-3-(2-ethoxy-pyridin-3-yl)-1-(2-methoxy-phenylsulfonyl)-2-oxo-2,3-dihydro-1H-indol-3-yl ester, Trifluoressigsäure-Salz

ESI-MS [M+H⁺] = 684 (CI-Isotopenmuster)

### BEISPIEL 24:

### (±)-4-(1-Methyl-piperidin-4-yl)-piperazin-1-carbonsäure1-phenylsulfonyl-5-chlor-3-(2-ethoxy-pyridin-3-yl)-2-oxo-2,3-dihydro-1H-indol-3-yl ester, Trifluoressigsäure-Salz

ESI-MS [M+H⁺] = 654 (CI-Isotopenmuster)

### BEISPIEL 25:

### (±)-4-(4-Methyl-piperazin-1-yl)-piperidin-1-carbonsäure5-chlor-1-(2,4-dimethoxyphenylsulfonyl)-3-(2-ethoxy-pyridin-3-yl)-2-oxo-2,3-dihydro-1H-indol-3-yl ester, Trifluoressigsäure-Salz

ESI-MS [M+H⁺] = 714 (CI-Isotopenmuster)

### BEISPIEL 26:

### (±)-4-(4-Methyl-piperazin-1-yl)-piperidin-1-carbonsäure5-chlor-3-(2-ethoxy-pyridin-3-yl)-1-(2-methoxy-phenylsulfonyl)-2-oxo-2,3-dihydro-1H-indol-3-yl ester, Trifluoressigsäure-Salz

ESI-MS [M+H⁺] = 684 (CI-Isotopenmuster)

### BEISPIEL 27:

### (±)-4-(4-Methyl-piperazin-1-yl)-piperidin-1-carbonsäure1-phenylsulfonyl-5-chlor-3-(2-ethoxy-pyridin-3-yl)-2-oxo-2,3-dihydro-1H-indol-3-yl ester, Trifluoressigsäure-Salz

ESI-MS [M+H⁺] = 654 (CI-Isotopenmuster)

### 11.3 Herstellung der racemischen Verbindungen der Formel I, worin X¹ für CH₂ steht (Beispiele 28 bis 33)

### BEISPIEL 28:

### (±)-5-Chlor-1-(2,4-dimethoxy-phenylsulfonyl)-3-(2-ethoxy-pyridin-3-yl)-3-{2-[4-(1-methyl-piperidin-4-yl)-piperazin-1-yl]-2-oxo-ethyl}-1,3-dihydroindol-2-on, Trifluoressigsäure-Salz

### 28.1 2-[5-Chlor-3-(2-ethoxy-pyridin-3-yl)-2-oxo-2,3-dihydro-1H-indol-3-yl]-malonsäuredimethylester

Zu einer auf 10 °C gekühlten Suspension von 3.56 g (89 mmol) Natriumhydrid (60% Dispersion in Mineralöl) in 150 ml Dimethylformamid tropfte man langsam 11.2 ml (98 mmol) Dimethylmalonat und rührte anschließend 30 Minuten bei Raumtemperatur. Danach gab man 9.6 g (30 mmol) 3,5-Dichlor-3-(2-ethoxy-pyridin-3-yl)-1,3-dihydroindol-2-on aus Beispiel 1.1 portionsweise zu und rührte 15 min bei Raumtemperatur. Man rührte die Umsetzung zur Aufarbeitung in kalte 1N HCl ein und versetzte mit Dichlormethan. Man trennte die Phasen getrennt und extrahierte die wässrige Phase mit Dichlormethan. Die vereinigte organische Phase wurde mit Wasser und gesättigter Natriumchlorid-Lösung gewaschen, über Magnesiumsulfat getrocknet und das Lösungsmittel im Vakuum entfernt. Man kristallisierte den Rückstand aus Dichlormethan / Pentan um. Man erhielt 9.01 g der Titelverbindung als gelblichen Feststoff.
ESI-MS [M+H⁺] = 419 (CI-Isotopenmuster).

### 28.2 [5-Chlor-3-(2-ethoxy-pyridin-3-yl)-2-oxo-2,3-dihydro-1H-indol-3-yl]-essigsäuremethylester

Verseifung: Zu einer Lösung von 9.00 g (21.5 mmol) 2-[5-Chlor-3-(2-ethoxy-pyridin-3-yl)-2-oxo-2,3-dihydro-1H-indol-3-yl]-malonsäuredimethylester in 9 ml Ethanol gab man 90 ml 2 N Natronlauge und rührte 1 Stunde bei Raumtemperatur. Man goss unter Rühren den Ansatz in eiskalte 1 N Salzsäure und extrahierte mehrmals mit Dichlormethan. Die vereinigte organische Phase wurde mit Wasser und gesättigter Natriumchlorid-Lösung gewaschen, über Magnesiumsulfat getrocknet und das Lösungsmittel im Vakuum entfernt. Man trocknete den daraus resultierende gelblichen Feststoff (8.41 g Diastereomerengemisch) im Vakuumtrockenschrank bei 40 °C. Decarboxylierung: Man erwärmte das vorstehend erhaltene Verseifungsprodukt (8.41 g) in einem mit Stickstoff inertisierten Einhalskolben auf 150 °C. Nachdem die CO₂-Entwicklung beendet war, ließ man den Reaktionsansatz auf Raumtemperatur abkühlen und verrührte den Rückstand mit Methanol. Es bildete sich ein Kristallisat, das im Kühlschrank über Nacht aufbewahrt wurde. Man erhielt 5.25 g der Titelverbindung als gelblichen Feststoff.
ESI-MS [M+H⁺] = 361 (Cl-Isotopenmuster).

### 28.3 [5-Chlor-3-(2-ethoxy-pyridin-3-yl)-2-oxo-2,3-dihydro-1H-indol-3-yl]-essigsäure

Zu einer Lösung von 1.96 g (5.43 mmol) [5-Chlor-3-(2-ethoxy-pyridin-3-yl)-2-oxo-2,3-dihydro-1H-indol-3-yl]-essigsäuremethylester in 10 ml Ethanol gab man 20 ml Wasser sowie 10 ml 2 N Natronlauge. Man rührte die Reaktionsmischung 18 Stunden bei Raumtemperatur. Zur Aufarbeitung wurde der Ansatz mit 1 N Salzsäure auf pH 5 gestellt und anschließend am Rotationsverdampfer eingeengt. Man nahm den Rückstand in Toluol auf, engte erneut bis zur Trockene ein und trocknete den Rückstand im Vakuumtrockenschrank. Man erhielt 3.75 g rohes Produkt, welches man ohne weitere Reinigung in der nächsten Stufe einsetzte.
ESI-MS [M+H⁺] = 347 (Cl-Isotopenmuster).

### 28.4 5-Chlor-3-(2-ethoxy-pyridin-3-yl)-3-{2-[4-(1-methyl-piperidin-4-yl)-piperazin-1-yl]-2-oxo-ethyl}-1,3-dihydroindol-2-on

Zu einer Lösung von 1.00 g [5-Chlor-3-(2-ethoxy-pyridin-3-yl)-2-oxo-2,3-dihydro-1H-indol-3-yl]-essigsäure in 10 ml Dimethylformamid gab man 195 mg (1.44 mmol) 1-Hydroxy-benzotriazol sowie 276 mg (1.44 mmol) N-(3-Dimethylaminopropyl)-N'-ethyl-carbodiimid-hydrochlorid, rührte 15 Minuten und gab danach 277 mg (1.51 mmol) 1-(1-Methylpiperidin-4-yl)-piperazin und 1.00 ml (7.41 mmol) Triethylamin. Man rührte die Reaktionsmischung über Nacht bei Raumtemperatur. Man versetzte den Reaktionsansatz mit Wasser und extrahierte mehrmals mit Essigester. Man extrahierte die vereinigte organische Phase mit Wasser und gesättigter Natriumchlorid-Lösung, trocknete über Magnesiumsulfat und engte das Lösungsmittel im Vakuum ein. Der Rückstand wurde chromatographisch über Kieselgel (12 g Redisep-Kartusche, Laufmittel-Gradient von 10 bis 70% Methanol in Dichlormethan) gereinigt. Man erhielt 311 mg der Titelverbindung als weißen Schaum.
ESI-MS [M+H⁺] = 512 (CI-Isotopenmuster).

### 28.5 5-Chlor-1-(2,4-dimethoxy-phenylsulfonyl)-3-(2-ethoxy-pyridin-3-yl)-3-{2-[4-(1-methylpiperidin-4-yl)-piperazin-1-yl]-2-oxo-ethyl}-1,3-dihydroindol-2-on, Trifluoressigsäure-Salz

Zu einer Lösung von 70 mg (0.14 mmol) 5-Chlor-3-(2-ethoxy-pyridin-3-yl)-3-{2-[4-(1-methyl-piperidin-4-yl)-piperazin-1-yl]-2-oxo-ethyl}-1,3-dihydroindol-2-on in 2 ml Dimethylformamid gab man unter Stickstoffatmosphäre und unter Kühlung durch ein Eisbad 6.6 mg (0.16 mmol) Natriumhydrid (60% Dispersion in Mineralöl). Man rührte die Reaktionsmischung 10 min bei 0 °C, gab danach 39 mg (0.16 mmol) 2,4-Dimethoxy-benzolsulfonylchlorid zu und rührte 30 min. bei Raumtemperatur nach. Man goss die Reaktionsmischung in Eiswasser und extrahierte anschließend mit Essigester. Man wusch die organische Phase mit gesättigter Natriumchlorid-Lösung, trocknete über Magnesiumsulfat und verdampfte das Lösungsmittel. Der Rückstand wurde durch präparative HPLC (Prontosil Prep 2012, C18, 125x20 mm, 5µm, Gradient von 10% bis 100% Acetonitril in Wasser, 0.1 % Trifluoressigsäure) gereinigt. Man erhielt: 45 mg der Titelverbindung als weißenFeststoff.
ESI-MS [M+H⁺] = 712 (Cl-Isotopenmuster)
¹H-NMR ([d₆]-DMSO, 500 MHz) δ [ppm] = 10.25 (1H), 8.10 (1H), 7.90 (2H), 7.80 (1H), 7.35 (1H), 7.20 (1H), 7.00 (1H), 6.65 (2H), 4.10 (2H), 3.95 (2H), 3.85 (3H), 3.60 (3H), 3.55 (2H), 2.90-3.50 (11H), 2.75 (3H), 2.25 (2H), 1.90 (2H), 1.00 (3H).

Die Verbindungen der Formel I, worin X¹ für CH₂ steht, gemäß den Beispielen 29 bis 33 können unter Verwendung der entsprechenden Sulfonylchloride der Formel VII sowie Amine der Formel X analog zum Herstellungsverfahren des Beispiels 28 hergestellt werden.

### BEISPIEL 29:

### (±)-1-Phenylsulfonyl-5-chlor-3-(2-ethoxy-pyridin-3-yl)-3-{2-[4-(1-methyl-piperidin-4-yl)-piperazin-1-yl]-2-oxo-ethyl}-1,3-dihydroindol-2-on, Trifluoressigsäure-Salz

ESI-MS [M+H⁺] = 652 (Cl-Isotopenmuster)

### BEISPIEL 30:

### (±)-5-Chlor-1-(2,4-dimethoxy-phenylsulfonyl)-3-(2-ethoxy-pyridin-3-yl)-3-{2-[4-(4-methyl-piperazin-1-yl)-piperidin-1-yl]-2-oxo-ethyl}-1,3-dihydroindol-2-on, Trifluoressigsäure-Salz

ESI-MS [M+H⁺] = 712 (Cl-Isotopenmuster)

BEISPIEL 31:

### (±)-1-Phenylsulfonyl-5-chlor-3-(2-ethoxy-pyridin-3-yl)-3-{2-[4-(4-methyl-piperazin-1-yl)-piperidin-1-yl]-2-oxo-ethyl}-1,3-dihydroindol-2-on, Trifluoressigsäure-Salz

ESI-MS [M+H⁺] = 652 (Cl-Isotopenmuster)

### BEISPIEL 32:

### (±)-5-Chlor-1-(2,4-dimethoxy-phenylsulfonyl)-3-(2-ethoxy-pyridin-3-yl)-3-{2-[4-(4-ethylpiperazin-1-yl)-piperidin-1-yl]-2-oxo-ethyl}-1,3-dihydroindol-2-on, Trifluoressigsäure-Salz

ESI-MS [M+H⁺] = 726 (Cl-Isotopenmuster)

### BEISPIEL 33:

### (±)-1-Phenylsulfonyl-5-ch lor-3-(2-ethoxy-pyridin-3-yl)-3-{2-[4-(4-ethyl-piperazin-1-yl)-piperidin-1-yl]-2-oxo-ethyl}-1,3-dihydroindol-2-one, Trifluoressigsäure-Salz

ESI-MS [M+H⁺] = 666 (Cl-Isotopenmuster)

### III. Herstellung der chiralen Verbindungen der Formel I

Die Trennung von racemischen Verbindungen der Formel I kann beispielsweise durch Separation auf einer präparativen chiralen Säule erfolgen.

### BEISPIEL 1A und BEISPIEL 1B

### Racematspaltung von (±)-4-(1-Methyl-piperidin-4-yl)-piperazin-1-carbonsäure [5-chlor-1-(2,4-dimethoxy-phenylsulfonyl)-3-(2-ethoxy-pyridin-3-yl)-2-oxo-2,3-dihydro-1H-indol-3-yl]-amid

100 mg racemisches 4-(1-Methyl-piperidin-4-yl)-piperazin-1-carbonsäure [5-chlor-1-(2,4-dimethoxy-phenylsulfonyl)-3-(2-ethoxy-pyridin-3-yl)-2-oxo-2,3-dihydro-1H-indol-3-yl]-amid aus Beispiel 1 wurden über eine chirale präparative Säule (Chiralcell OD, Fluss 55 ml /min) mit *n*-Heptan / Ethanol (7 : 3) als Elutionsmittel getrennt. Ausbeute: 33 mg des linksdrehenden Enantiomers (Beispiel 1A) und 27 mg des rechtsdrehenden Enantiomers (Beispiel 1 B).

### BEISPIEL 1A:

### (-) 4-(1-Methyl-piperidin-4-yl)-piperazin-1-carbonsäure [5-chlor-1-(2,4-dimethoxyphenylsulfonyl)-3-(2-ethoxy-pyridin-3-yl)-2-oxo-2,3-dihydro-1H-indol-3-yl]-amid

ESI-MS [M+H⁺] = 713 (Cl-Isotopenmuster)
¹H-NMR ([d₆]-DMSO, 500 MHz) δ [ppm] = 8.10 (1H), 7.85 (1H), 7.70 (1H), 7.55 (2H), 7.35 (2H), 6.95 (1H), 6.65 (2H), 4.20 (2H), 3.85 (3H), 3.45 (3H), 3.20 (4H), 2.75 (2H), 2.35 (4H), 2.10 (4H), 1.80 (2H), 1.65 (2H), 1.35 (2H), 1.15 (3H).
HPLC (Chiralcel OD 0.46 cm x 25 cm ; n-Heptan / Ethanol 7:3) R_{f} = 7.4 min
[α]²⁰_{D} = - 16 (c 0.1, CHCl₃)

BEISPIEL 1B:

### (+) 4-(1-Methyl-piperidin-4-yl)-piperazin-1-carbonsäure [5-chlor-1-(2,4-dimethoxyphenylsulfonyl)-3-(2-ethoxy-pyridin-3-yl)-2-oxo-2,3-dihydro-1H-indol-3-yl]-amid

ESI-MS [M+H⁺] = 713 (Cl-Isotopenmuster)
¹H-NMR ([d₆]-DMSO, 500 MHz) δ [ppm] = 8.10 (1H), 7.85 (1H), 7.70 (1H), 7.55 (2H), 7.35 (2H), 6.95 (1H), 6.65 (2H), 4.20 (2H), 3.85 (3H), 3.45 (3H), 3.20 (4H), 2.75 (2H), 2.35 (4H), 2.10 (4H), 1.80 (2H), 1.65 (2H), 1.35 (2H), 1.15 (3H).
HPLC (Chiralcel OD 0.46 cm x 25 cm ; n-Heptan / Ethanol 7:3) R_{f} = 20.0 min
[α]²⁰_{D} = + 12 (c 0.1, CHCl₃)

In analoger Weise lassen sich die Racemate der BEISPIELE 4, 5 und 8 unter Erhalt der entsprechenden Enantiomere trennen.

### BEISPIEL 4B:

### (+) 4-(1-Ethyl-piperidin-4-yl)-piperazin-1-carbonsäure [5-chlor-1-(2,4-dimethoxyphenylsulfonyl)-3-(2-ethoxy-pyridin-3-yl)-2-oxo-2,3-dihydro-1H-indol-3-yl]-amid

ESI-MS [M+H⁺] = 727 (CI-Isotopenmuster)
¹H-NMR ([d₆]-DMSO, 500 MHz) δ [ppm] = 8.10 (1H), 7.85 (1H), 7.70 (1H), 7.55 (2H), 7.35 (2H), 6.95 (1H), 6.65 (2H), 4.20 (2H), 3.85 (3H), 3.45 (3H), 3.20 (4H), 2.85 (2H), 2.35 (4H), 2.30 (2H), 2.10 (1H), 1.80 (2H), 1.65 (2H), 1.35 (2H), 1.10 (3H), 0.95 (3H).
HPLC (Chiralcel OD 0.46 cm x 25 cm ; n-Heptan / Ethanol 7:3) R_{f} = 17.9 min
[α]²⁰_{D} = +12 (c 0.1, CHCl₃)

### BEISPIEL 5A:

### (-) 4-(4-Methyl-piperazin-1-yl)-piperidin-1-carbonsäure [5-chlor-1-(2,4-dimethoxyphenylsulfonyl)-3-(2-ethoxy-pyridin-3-yl)-2-oxo-2,3-dihydro-1H-indol-3-yl]-amid

ESI-MS [M+H⁺] = 713 (CI-Isotopenmuster)
¹H-NMR ([d₆]-DMSO, 500 MHz) δ [ppm] = 8.10 (1H), 7.90 (1H), 7.70 (1H), 7.60 (1H), 7.55 (1H), 7.35 (2H), 6.95 (1H), 6.65 (2H), 4.25 (2H), 3.85 (3H), 3.80 (2H), 3.45 (3H), 2.65 (2H), 2.40 (4H), 2.30 (4H), 2.15 (3H), 1.60 (2H), 1.15 (5H).
HPLC (Chiralcel OD 0.46 cm x 25 cm ; n-Heptan / Ethanol 7:3) R_{f} = 7.6 min
[α]²⁰_{D} = - 14 (c 0.1, CHCl₃)

### BEISPIEL 5B:

### (+) 4-(4-Methyl-piperazin-1-yl)-piperidin-1-carbonsäure [5-chlor-1-(2,4-dimethoxyphenylsulfonyl)-3-(2-ethoxy-pyridin-3-yl)-2-oxo-2,3-dihydro-1H-indol-3-yl]-amid

ESI-MS [M+H⁺] = 713 (CI-Isotopenmuster)
¹H-NMR ([d₆]-DMSO, 500 MHz) δ [ppm] = 8.10 (1H), 7.90 (1H), 7.70 (1H), 7.60 (1H), 7.55 (1H), 7.35 (2H), 6.95 (1H), 6.65 (2H), 4.25 (2H), 3.85 (3H), 3.80 (2H), 3.45 (3H), 2.65 (2H), 2.40 (4H), 2.30 (4H), 2.15 (3H), 1.60 (2H), 1.15 (5H).
HPLC (Chiralcel OD 0.46 cm x 25 cm ; n-Heptan / Ethanol 7:3) R_{f} = 12.2 min
[α]²⁰_{D} = + 9 (c 0.1, CHCl₃)

### BEISPIEL 8B:

### (+) 4-(4-Ethyl-piperazin-1-yl)-piperidin-1-carbonsäure [5-chlor-1-(2,4-dimethoxyphenylsulfonyl)-3-(2-ethoxy-pyridin-3-yl)-2-oxo-2,3-dihydro-1H-indol-3-yl]-amid

ESI-MS [M+H⁺] = 727 (CI-Isotopenmuster)
¹H-NMR ([d₆]-DMSO, 500 MHz) δ [ppm] = 8.10 (1H), 7.90 (1H), 7.70 (1H), 7.60 (1H), 7.55 (1H), 7.35 (2H), 6.95 (1H), 6.65 (2H), 4.20 (2H), 3.85 (3H), 3.80 (2H), 3.45 (3H), 2.65 (2H), 2.25-2.50 (11H), 1.65 (2H), 1.15 (5H), 0.95 (3H).
HPLC (Chiralcel OD 0.46 cm x 25 cm ; n-Heptan / Ethanol 7:3) R_{f} = 11.5 min
[α]²⁰_{D} = + 20 (c 0.1, CHCl₃)

### IV. BESTIMMUNG DER BIOLOGISCHEN AKTIVITÄT

### 1. Vasopressin V1b Rezeptorbindungstest:

### Substanzen:

Die Testsubstanzen wurden in einer Konzentration von 10⁻² M in DMSO gelöst und in DMSO auf 5x10⁻⁴ M bis 5x10⁻⁹ M weiter verdünnt. Diese DMSO-Vorverdünnungsreihe wurde mit Testpuffer 1:10 verdünnt. Im Testansatz wurde die Substanzkonzentration nochmals 1:5 verdünnt (2 % DMSO im Ansatz).

### Membran-Präparation:

CHO-K1 Zellen mit stabil exprimiertem humanem Vasopressin V1 b Rezeptor (Klon 3H2) wurden abgeerntet und in 50 mM Tris-HCl und in Gegenwart von Proteaseinhibitoren (Roche complete Mini # 1836170) mit einem Polytron Homogenizer auf mittlerer Stellung 2x10 Sekunden homogenisiert und anschließend 1h bei 40.000 x g abzentrifugiert. Das Membranpellet wurde nochmals wie beschrieben homogenisiert und zentrifugiert und anschließend in 50 mM Tris-HCl, pH 7,4 aufgenommen, homogenisiert und in Aliquots bei -190°C in flüssigem Stickstoff eingefroren aufbewahrt.

### Bindungstest:

Der Bindungstest wurde in Anlehnung an die Methode von Tahara et al. (Tahara A et al., Brit. J. Pharmacol. 125, 1463-1470 (1998)) durchgeführt.
Inkubationspuffer war: 50 mM Tris, 10 mM MgCl₂, 0.1% BSA, pH 7.4.
Im Testansatz (250 µl) wurden Membranen (50 µg/ml Protein in Inkubationspuffer) von CHO-K1-Zellen mit stabil exprimierten humanen V1b Rezeptoren (Zelllinie hV1b_3H2_CHO) mit 1,5 nM ³H-AVP (8-Arg-Vasopressin, PerkinElmer #18479) in Inkubationspuffer (50 mM Tris, 10 mM MgCl₂, 0.1% BSA, pH 7.4) (totale Bindung) oder zusätzlich mit steigenden Konzentrationen an Testsubstanz (Verdrängungsexperiment) inkubiert. Die unspezifische Bindung wurde mit 1 M AVP (Bachem # H1780) bestimmt. Alle Bestimmungen wurden als Dreifachbestimmungen durchgeführt. Nach Inkubation (60 Minuten bei Raumtemperatur), wurde der freie Radioligand mittels Vakuumfiltration (Skatron cell harvester 7000) über Wathman GF/B Glasfaserfiltermatten abfiltriert und die Filter in Szintillationsgefäße überführt. Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

### Auswertung:

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)). Der Kd-Wert von ³H-AVP zu den rekombinanten humanen V1b-Rezeptoren beträgt 0,4 nM und wurde zur Bestimmung des Ki-Wertes herangezogen.

### 2. Vasopressin V1a Rezeptorbindungstest:

### Substanzen:

Die Testsubstanzen wurden in einer Konzentration von 10⁻² M in DMSO gelöst. Die weitere Verdünnung dieser DMSO-Lösungen erfolgte in Inkubationspuffer (50 mM Tris, 10 mM MgCl₂, 0.1 % BSA, pH 7,4).

### Membran-Präparation:

CHO-K1 Zellen mit stabil exprimiertem humanem Vasopressin- V1a Rezeptor (Klon 5) wurden abgeerntet und in 50 mM Tris-HCl und in Gegenwart von Proteaseinhibitoren (Roche complete Mini # 1836170) mit einem Polytron Homogenizer auf mittlerer Stellung 2x10 Sekunden homogenisiert und anschließend 1 h bei 40.000 x g abzentrifugiert. Das Membranpellet wurde nochmals wie beschrieben homogenisiert und zentrifugiert und anschließend in 50 mM Tris-HCl, pH 7,4 aufgenommen, homogenisiert und in Aliquots bei -190°C in flüssigem Stickstoff eingefroren aufbewahrt.

### Bindungstest:

Der Bindungstest wurde in Anlehnung an die Methode von Tahara et al. (Tahara A et al., Brit. J. Pharmacol. 125, 1463-1470 (1998)) durchgeführt.
Inkubationspuffer war: 50 mM Tris, 10 mM MgCl₂, 0.1% BSA, pH 7.4.
Im Testansatz (250 µl) wurden Membranen (20 µg/ml Protein in Inkubationspuffer) von CHO-K1-Zellen mit stabil exprimierten humanen V1a Rezeptoren (Zelllinie hV1a_5_CHO) mit 0.04 nM ¹²⁵I-AVP (8-Arg-Vasopressin, NEX 128) in Inkubationspuffer (50 mM Tris, 10 mM MgCl₂, 0.1% BSA, pH 7.4) (totale Bindung) oder zusätzlich mit steigenden Konzentrationen an Testsubstanz (Verdrängungsexperiment) inkubiert. Die unspezifische Bindung wurde mit 1 µM AVP (Bachem # H1780) bestimmt. Dreifachbestimmungen wurden durchgeführt.
Nach Inkubation (60 Minuten bei Raumtemperatur), wurde der freie Radioligand mittels Vakuumfiltration (Skatron cell harvester 7000) über Wathman GF/B Glasfaserfiltermatten abfiltriert und die Filter in Szintillationsgefäße überführt.
Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

### Auswertung:

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)). Der Kd-Wert von ¹²⁵I -AVP zu den rekombinanten hV1a-Rezeptoren wurde in Sättigungsexperimenten bestimmt. Ein Kd-Wert von 1,33 nM wurde zur Bestimmung des Ki-Wertes herangezogen.

### 3. Vasopressin V2 Rezeptorbindungstest:

### Substanzen:

Die Testsubstanzen wurden in einer Konzentration von 10⁻² M in DMSO gelöst. Die weitere Verdünnung dieser DMSO-Lösung erfolgte in Inkubationspuffer (50 mM Tris, 10 mM MgCl₂, 0.1 % BSA, pH 7,4).

### Membran-Präparation:

CHO-K1 Zellen mit stabil exprimiertem humanem Vasopressin V2 Rezeptor (Klon 23) wurden abgeerntet und in 50 mM Tris-HCl und in Gegenwart von Proteaseinhibitoren (Roche complete Mini # 1836170) mit einem Polytron Homogenizer auf mittlerer Stellung 2x10 Sekunden homogenisiert und anschließend 1 h bei 40.000 x g abzentrifugiert. Das Membranpellet wurde nochmals wie beschrieben homogenisiert und zentrifugiert und anschließend in 50 mM Tris-HCl, pH 7,4 aufgenommen, homogenisiert und in Aliquots bei -190°C in flüssigem Stickstoff eingefroren aufbewahrt.

### Bindungstest:

Der Bindungstest wurde in Anlehnung an die Methode von Tahara et al. (Tahara A et al., Brit. J. Pharmacol. 125, 1463-1470 (1998)) durchgeführt.
Inkubationspuffer war: 50 mM Tris, 10 mM MgCl₂, 0.1% BSA, pH 7.4.
Im Testansatz (250 µl) wurden Membranen (50 µg/ml Protein in Inkubationspuffer) von CHO-Kl-Zellen mit stabil exprimierten humanen V2 Rezeptoren (Zelllinie hV2_23_CHO) mit 1-2 nM ³H-AVP (8-Arg-Vasopressin, PerkinElmer #18479) in Inkubationspuffer (50 mM Tris, 10 mM MgCl₂, 0.1% BSA, pH 7.4) (totale Bindung) oder zusätzlich mit steigenden Konzentrationen an Testsubstanz (Verdrängungsexperiment) inkubiert. Die unspezifische Bindung wurde mit 1 µM AVP (Bachem # H1780) bestimmt. Dreifachbestimmungen wurden durchgeführt.
Nach Inkubation (60 Minuten bei Raumtemperatur), wurde der freie Radioligand mittels Vakuumfiltration (Skatron cell harvester 7000) über Wathman GF/B Glasfaserfiltermatten abfiltriert und die Filter in Szintillationsgefäße überführt.
Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

### Auswertung:

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)). Der Kd-Wert von ³H-AVP zu den rekombinanten hV2-Rezeptoren beträgt 2,4 nM und wurde zur Bestimmung des Ki-Wertes herangezogen.

### 4. Oxytozin-Rezeptorbindungstest

### Substanzen:

Die Substanzen wurden in einer Konzentration von 10⁻² M in DMSO gelöst und mit Inkubationspuffer (50 mM Tris, 10 mM MgCl₂, 0.1 % BSA, pH 7,4) verdünnt.

### Zellpräparation:

Konfluente HEK-293 Zellen mit transient exprimierenden rekombinanten humanen Oxytozinrezeptoren wurden bei 750 x g für 5 Minuten bei Raumtemperatur zentrifugiert. Der Rückstand wurde in eiskaltem Lysispuffer (50 mM Tris-HCl, 10 % Glycerin, pH 7,4 und Roche Complete Protease-Inhibitor) aufgenommen und 20 Minuten bei 4 °C einem osmotischen Schock unterworfen. Danach wurden die lysierten Zellen bei 750 x g für 20 Minuten bei 4°C zentrifugiert, der Rückstand in Inkubationspuffer aufgenommen und Aliquots von 10⁷ Zellen/ml hergestellt. Die Aliquots wurden bis zur Verwendung bei -80°C eingefroren.

### Bindungstest:

Am Tag des Versuches wurden die Zellen aufgetaut, mit Inkubationspuffer verdünnt und mit einer Multipette Combitip (Eppendorf, Hamburg) homogenisiert. Der Reaktionsansatz von 0,250 ml setzte sich zusammen aus 2 bis 5x10⁴ rekombinante Zellen, 3-4 nM ³H-Oxytozin (PerkinElmer, NET 858) in Anwesenheit von Testsubstanz (Hemmkurve) oder nur Inkubationspuffer (totale Bindung). Die unspezifische Bindung wurde mit 10⁻⁶ M Oxytozin (Bachem AG, H2510) bestimmt. Dreifachbestimmungen wurden angesetzt. Gebundener und freier Radioligand wurden getrennt durch Filtration unter Vakuum mit Whatman GF/B Glasfaserfilter mit Hilfe eines Skatron Cell Harvester 7000. Die gebundene Radioaktivität wurde durch Flüssigkeitszintillationsmessung in einem Tricarb Beta-Zählgerät, Modell 2000 oder 2200CA (Packard) bestimmt.

### Auswertung:

Die Bindungsparameter wurden durch nicht-lineare Regressionsanalyse berechnet (SAS), analog zum Programm LIGAND von Munson und Rodbard (Analytical Biochem 1980; 107: 220-239). Der Kd-Wert von ³H-Oxytozin zu den rekombinanten hOT-Rezeptoren beträgt 7.6 nM und wurde zur Bestimmung des Ki-Wertes herangezogen.

### 5. Bestimmung der mikrosomalen Halbwertszeit:

Die metabolische Stabilität der erfindungsgemäßen Verbindungen wurde in dem folgenden Test bestimmt.

Die Testsubstanzen werden in einer Konzentration von 0.5 µM wie folgt inkubiert:
In Mikrotiterplatten werden 0.5 µM Testsubstanz zusammen mit Lebermikrosomen verschiedener Spezies (von Ratte, Mensch oder andere Spezies) (0.25 mg mikrosomales Protein/ml) in 0.05M Kalium-Phosphatpuffer pH 7,4 bei 37 °C für 5 min vorinkubiert. Der Start der Reaktion erfolgt durch die Zugabe von NADPH (1 mg/mL). Nach 0, 5, 10, 15, 20 und 30 min werden 50 µl Aliquots entnommen und die Reaktion wird sofort mit dem gleichen Volumen an Acetonitril abgestoppt und runtergekühlt. Die Proben werden bis zur Analyse eingefroren. Mittels MSMS wird die verbliebene Konzentration an nicht abgebauter Testsubstanz bestimmt. Aus der Steigung der Kurve Signal Testsubstanz/Zeiteinheit wird die Halbwertszeit (T1/2) ermittelt, wobei die Halbwertszeit der Testsubstanz unter Annahme einer Kinetik erster Ordnung aus der zeitlichen Abnahme der Konzentration der Verbindung berechnet werden kann. Die mikrosomale Clearance (mCl) berechnet sich aus mCl= In2/T1/2 / (Gehalt an mikrosomalem Protein in mg/ml) x 1000 [ml/min/mg] (modifiziert nach Literaturstellen: Di, The Society for Biomoleculaur Screening, 2003, 453-462; Obach, DMD, 1999 vol 27. N 11, 1350-1359).

### 6. Methoden zur in vitro Bestimmung der Cytochrom P450 (CYP) Inhibierung

### Lumineszenzsubstrate für 2C9 und 3A4:

0.4 mg/ml humane Lebermikrosomen werden 10 min mit den zu untersuchenden Testsubstanzen (0-20 µM), den CYP-spezifischen Substraten, in 0.05 M Kaliumphosphatpuffer pH 7,4 bei 37 °C vorinkubiert. Das Cyp-spezifische Substrat für CYP 2C9 ist Luciferin H, für CYP 3A4 Luciferin BE. Die Reaktion wird durch Hinzufügen von NADPH gestartet. Nach 30 min Inkubation bei RT wird das Luciferin Detektionsreagenz hinzugefügt, und das entstandene Lumineszenzsignal gemessen (modifiziert nach Literaturstelle: Promega, Technical Bulletin P450-GLO^{™} Assays).

### Midazolam CYP 3A4 Time dependent Inhibition

Der Test besteht aus 2 Teilen. Einmal wird die Testsubstanz mit den Lebermikrosomen vorinkubiert (mit NADPH = Prä-Inkubation, danach Zugabe des Substrates, im 2 .Teil wird das Substrat und die Testsubstanz gleichzeitig zugegeben = Co-Inkubation.

### Prä-Inkubation:

0.05 mg/ml mikrosomales Protein (humane Lebermikrosomen) werden mit 0-10 µM (bzw 50 µM) Testsubstanz in 50 mM Kaliumphosphatpuffer 5 Min vorinkubiert. Die Reaktion wird mit NADPH gestartet. Nach 30 min werden 4 µM Midazolam (Endkonzentration) hinzugefügt und für weitere 10 Min inkubiert. 75 µl der Reaktionslösung werden nach 10 min entnommen und mit 150 µl Acetonitrillösung abgestoppt.

### Co-Inkubation:

0.05 mg/ml mikrosomales Protein (humane Lebermikrosomen) werden mit 4 µM Midazolam (Endkonzentration) und 0-10 µM (bzw 50µM) Testsubstanz in 50 mM Kaliumphosphatpuffer 5 min vorinkubiert. Die Reaktion wird mit NADPH gestartet. 75 µl der Reaktionslösung werden nach 10 min entnommen und mit 150 µl Acetonitrillösung abgestoppt. Die Proben werden bis zur MSMS-Analytik eingefroren (modifiziert nach Literaturstellen: Obdach, Journal of Pharmacology & Experimental Therapeutics, Vol 316, 1, 336-348, 2006; Walsky, Drug Metabolism and Disposition Vol 32, 6, 647-660, 2004).

### 7. Methode zur Bestimmung der Wasserlöslichkeit (in mg/ml)

Die Wasserlöslichkeit der erfindungsgemässen Verbindungen kann zum Beispiel nach der sogenannten "shake flask" Methode bestimmt werden (gemäss ASTM International: E 1148-02, Standard test methods for measurement of aqueous solubility, Book of Standards Volume 11.05*.).* Dabei wird ein Überschuss der festen Verbindung in eine Pufferlösung mit einem bestimmten pH-Wert (zum Beispiel Phosphatpuffer pH 7.4) gegeben und die entstehende Mischung geschüttelt oder gerührt, bis sich das Gleichgewicht eingestellt hat (typischerweise 24 oder 48 Stunden, manchmal auch bis zu 7 Tage). Anschließend wird der ungelöste Feststoff durch Filtrieren oder Zentrifugieren abgetrennt und die Konzentration der gelösten Verbindung durch UV-Spektroskopie oder HochdruckflüssigkeitsChromatographie (HPLC) anhand einer entsprechenden Kalibrierkurve bestimmt.

### 8. Ergebnisse

Die Ergebnisse der Rezeptor-Bindungsuntersuchungen sind als Rezeptorbindungskonstanten [Kᵢ(V1b)] bzw. Selektivitäten [Kᵢ(V1a)/ Kᵢ(V1b)] ausgedrückt. Die Ergebnisse der Untersuchung der metabolischen Stabilität sind als mikrosomale Clearance (mCl) angegeben.

In diesen Tests zeigen die erfindungsgemäßen Verbindungen sehr hohe Affinitäten für den V1b-Rezeptor (höchstens 100 nM, oder höchstens 10 nM, häufig < 1 nM). Außerdem zeigen die Verbindungen auch hohe Selektivitäten gegenüber dem V1a -Rezeptor und eine gute metabolische Stabilität, gemessen als mikrosomale Clearance.

Die Ergebnisse sind in Tabelle 2 aufgeführt. Die Nummern der Verbindungen beziehen sich auf die Synthesebeispiele.

**Tabelle 2**

| Beispiel | Kᵢ(h-V1b)* [nM] | Kᵢ(h-V1a)/Kᵢ(h-V1b)* | Humane mikrosomale Clearance [µl min⁻¹ mg⁻¹] |
|---|---|---|---|
| 1 | +++ | +++ | + |
| 3 | ++ | ++ | +++ |
| 13 | +++ | ++ | + |
| 14 | +++ | +++ | ++ |
| 15 | +++ | ++ | + |
| 16 | +++ | + | + |
| 17 | +++ | + | + |
| 24 | ++ | ++ | ++ |
| 28 | +++ | ++ | + |
| 29 | ++ | + | +++ |
| 32 | +++ | + | ++ |
| 1B | +++ | +++ | +++ |
| 4B | +++ | +++ | ++ |
| 5B | +++ | +++ | +++ |
| 8B | +++ | +++ | + |

| | | | |
|---|---|---|---|
| * h = human | | | |

**Schlüssel:**

| | Kᵢ(V1b) | Kᵢ(h-V1a)/Kᵢ(h-V1b) | Humane mikrosomale Clearance |
|---|---|---|---|
| + | > 10 - 100 nM | 10 - < 25 | > 75 - 100 µl min⁻¹ mg⁻¹ |
| ++ | 1 - 10 nM | 25 - 75 | 50 - 75 µl min⁻¹ mg⁻¹ |
| +++ | < 1 nM | > 75 | < 50 µl min⁻¹ mg⁻¹ |

## Patentansprüche

1. Verbindungen der Formel I 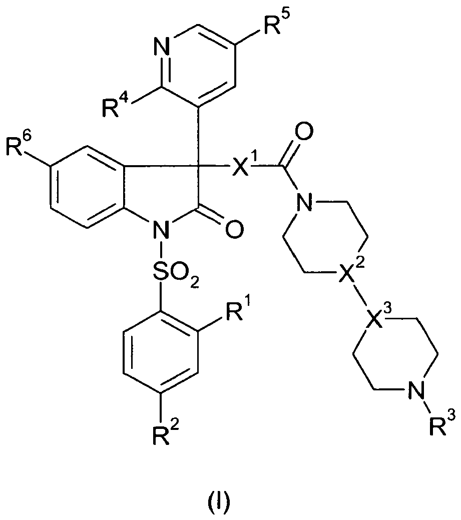 worin
R¹ für Wasserstoff, Methoxy oder Ethoxy steht;
R² für Wasserstoff oder Methoxy steht;
R³ für Wasserstoff, Methyl, Ethyl, n-Propyl oder Isopropyl steht;
R⁴ für Ethoxy oder Isopropoxy steht;
R⁵ für H oder Methyl steht;
R⁶ für Cl oder F steht;
X¹ für O, NH oder CH₂ steht; und
X² und X³ für N oder CH stehen, unter der Maßgabe, dass X² und X³ nicht gleichzeitig für N stehen;
sowie deren pharmazeutisch verträglichen Salze.

2. Verbindungen nach Anspruch 1, worin R¹ für Wasserstoff oder Methoxy steht.

3. Verbindungen nach Anspruch 1, worin R¹ und R² für Methoxy stehen.

4. Verbindungen nach einem der vorhergehenden Ansprüche, worin R³ für Wasserstoff, Methyl oder Ethyl steht.

5. Verbindungen nach einem der vorhergehenden Ansprüche, worin R⁴ für Ethoxy steht und R⁵ für H steht.

6. Verbindungen nach einem der Ansprüche 1 bis 4, worin R⁴ für Ethoxy steht und R⁵ für Methyl steht.

7. Verbindungen nach einem der Ansprüche 1 bis 4, worin R⁴ für Isopropoxy steht und R⁵ für H steht.

8. Verbindungen nach einem der vorhergehenden Ansprüche, worin R⁶ für Cl steht.

9. Verbindungen nach einem der Ansprüche 1 bis 7, worin R⁶ für F steht.

10. Verbindungen nach einem der vorhergehenden Ansprüche, worin X² für N steht und X³ für CH steht.

11. Verbindungen nach einem der Ansprüche 1 bis 9, worin X² für CH steht und X³ für N steht.

12. Verbindungen nach einem der vorhergehenden Ansprüche, worin
R¹ für Methoxy steht;
R² für Methoxy steht;
R³ für Methyl steht;
R⁴ für Ethoxy steht;
R⁵ für H steht;
R⁶ für Cl steht;
X¹ für NH steht;
X² für N steht; und
X³ für CH steht.

13. Verbindungen nach einem der Ansprüche 1 bis 11, worin
R¹ für Methoxy steht;
R² für Methoxy steht;
R³ für Methyl steht;
R⁴ für Ethoxy steht;
R⁵ für Methyl steht;
R⁶ für Cl steht;
X¹ für NH steht;
X² für N steht; und
X³ für CH steht.

14. Verbindungen nach einem der Ansprüche 1 bis 11, worin
R¹ für Methoxy steht;
R² für Methoxy steht;
R³ für Ethyl steht;
R⁴ für Ethoxy steht;
R⁵ für H steht;
R⁶ für Cl steht;
X¹ für NH steht;
X² für N steht; und
X³ für CH steht.

15. Verbindungen nach einem der Ansprüche 1 bis 11, worin
R¹ für Methoxy steht;
R² für Methoxy steht;
R³ für Methyl steht;
R⁴ für Ethoxy steht;
R⁵ für H steht;
R⁶ für Cl steht;
X¹ für NH steht;
X² für CH steht; und
X³ für N steht.

16. Verbindungen nach einem der vorhergehenden Ansprüche, wobei es sich um das (+)-Enantiomer in einer Enantiomerenreinheit von wenigstens 90 % ee handelt.

17. Verbindung nach einem der Ansprüche 1 bis 15, wobei es sich um das Racemat handelt.

18. Pharmazeutisches Mittel, enthaltend wenigstens eine Verbindung der Formel I gemäß der Definition in einem der vorhergehenden Ansprüche und/oder wenigstens ein pharmazeutisch verträgliches Salz davon und wenigstens einen pharmazeutisch verträglichen Träger.

19. Verwendung von Verbindungen der Formel I gemäß der Definition in einem der Ansprüche 1 bis 17 oder von pharmazeutisch verträglichen Salzen davon zur Herstellung eines Medikaments zur Behandlung und/oder Prophylaxe von Vasopressin-abhängigen Krankheiten.

20. Verwendung nach Anspruch 19, zur Herstellung eines Medikaments zur Behandlung und/oder Prophylaxe von Krankheiten, die ausgewählt sind unter Diabetes, Insulin-Resistenz, Enuresis nocturna, Inkontinenz, Krankheiten, bei denen Blutgerinnungsstörungen auftreten,
Hypertonie, pulmonaler Hypertonie, Herzinsuffizienz, Myocardinfarkt, koronarem Spasmus, instabiler Angina, PTCA (percutaneous transluminal coronary angioplastie), Ischämien des Herzens, Störungen des renalen Systems, Ödemen, renalem Vasospasmus, Nekrose des renalen Cortex, Hyponatriämie, Hypokaliämie, Schwartz-Bartter Syndrom, Störungen des Gastrointestinaltraktes, gastritischem Vasospasmus, Hepatozirrhose, Magen- und Darmulkus, Emesis, auftretender Emesis bei der Chemotherapie, Reisekrankheit,
affektiven Störungen,
Angststörungen, stressabhängigen Angststörungen, Gedächtnisleistungsstörungen, Morbus Alzheimer,
Psychosen, psychotischen Störungen,
Cushing-Syndrom, sonstigen stress-abhängigen Krankheiten, Schlafstörungen,
depressiven Erkrankungen, vorzugsweise childhood onset mood disorders, vasomotorischen Symptomen, thermoregulatorischen Fehlfunktionen, Drogen-, Arzneimittel- und/oder durch sonstige Faktoren vermittelten Abhängigkeiten; von Stress bedingt durch den Entzug von einem oder mehreren die Abhängigkeit vermittelnden Faktoren; und/oder von Stress-induzierten Rückfällen in die Drogen-, Arzneimittel- und/oder durch sonstige Faktoren vermittelte Abhängigkeiten,
Schizophrenie, und Psychose,
und/oder zur Verzögerung der Miktion.

## Claims

1. Compound of the formula I 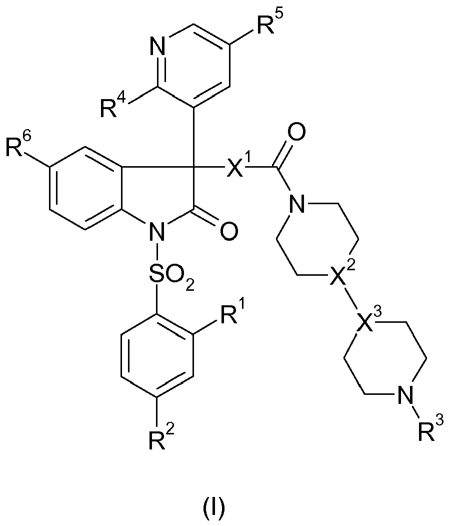 in which
R¹ is hydrogen, methoxy or ethoxy;
R² is hydrogen or methoxy;
R³ is hydrogen, methyl, ethyl, n-propyl or isopropyl;
R⁴ is ethoxy or isopropoxy;
R⁵ is H or methyl;
R⁶ is Cl or F;
X¹ is O, NH or CH₂; and
X² and X³ are N or CH, with the proviso that X² and X³ are not simultaneously N;
and their pharmaceutically acceptable salts.

2. Compound according to claim 1, in which R¹ is hydrogen or methoxy.

3. Compound according to claim 1, in which R¹ and R² are methoxy.

4. Compound according to any of the preceding claims, in which R³ is hydrogen, methyl or ethyl.

5. Compound according to any of the preceding claims, in which R⁴ is ethoxy and R⁵ is H.

6. Compound according to any of claims 1 to 4, in which R⁴ is ethoxy and R⁵ is methyl.

7. Compound according to any of claims 1 to 4, in which R⁴ is isopropoxy and R⁵ is H.

8. Compound according to any of the preceding claims, in which R⁶ is Cl.

9. Compound according to any of claims 1 to 7, in which R⁶ is F.

10. Compound according to any of the preceding claims, in which X² is N and X³ is CH.

11. Compound according to any of claims 1 to 9, in which X² is CH and X³ is N.

12. Compound according to any of the preceding claims, in which
R¹ is methoxy;
R² is methoxy;
R³ is methyl;
R⁴ is ethoxy;
R⁵ is H;
R⁶ is Cl;
X¹ is NH;
X² is N; and
X³ is CH.

13. Compound according to any of claims 1 to 11, in which
R¹ is methoxy;
R² is methoxy;
R³ is methyl;
R⁴ is ethoxy;
R⁵ is methyl;
R⁶ is Cl;
X¹ is NH;
X² is N; and
X³ is CH.

14. Compound according to any of claims 1 to 11, in which
R¹ is methoxy;
R² is methoxy;
R³ is ethyl;
R⁴ is ethoxy;
R⁵ is H;
R⁶ is Cl;
X¹ is NH;
X² is N; and
X³ is CH.

15. Compound according to any of claims 1 to 11, in which
R¹ is methoxy;
R² is methoxy;
R³ is methyl;
R⁴ is ethoxy;
R⁵ is H;
R⁶ is Cl;
X¹ is NH;
X² is CH; and
X³ is N.

16. Compound according to any of the preceding claims, which is the (+) enantiomer in an enantiomeric purity of at least 90% ee.

17. Compound according to any of claims 1 to 15, which is the racemate.

18. Pharmaceutical composition comprising at least one compound of the formula I as defined in any of the preceding claims and/or at least one pharmaceutically acceptable salt thereof and at least one pharmaceutically acceptable carrier.

19. Use of compounds of the formula I as defined in any of claims 1 to 17 or of pharmaceutically acceptable salts thereof for the manufacture of a medicament for the treatment and/or prophylaxis of vasopressin-dependent diseases.

20. Use according to claim 19, for the manufacture of a medicament for the treatment and/or prophylaxis of diseases selected from diabetes, insulin resistance, nocturnal enuresis, incontinence, diseases in which impairments of blood clotting occur,
hypertension, pulmonary hypertension, heart failure, myocardial infarction, coronary spasm, unstable angina, PTCA (percutaneous transluminal coronary angioplasty), ischemias of the heart, impairments of the renal system, edemas, renal vasospasm, necrosis of the renal cortex, hyponatremia, hypokalemia, Schwartz-Bartter syndrome, impairments of the gastrointestinal tract, gastritic vasospasm, hepatocirrhosis, gastric and intestinal ulcers, emesis, emesis occurring during chemotherapy, travel sickness, affective disorders,
anxiety disorders, stress-dependent anxiety disorders,
memory impairments, Alzheimer's disease, psychoses, psychotic disorders,
Cushing's syndrome, other stress-dependent diseases,
sleep disorders,
depressive disorders, preferably childhood onset mood disorders,
vasomotor symptoms, thermoregulatory dysfunctions, drug or pharmaceutical dependencies and/or dependencies mediated by other factors; of stress caused by withdrawal of one or more factors mediating the dependence; and/or of stress-induced relapses into drug or pharmaceutical dependencies and/or dependencies mediated by other factors, schizophrenia, and psychosis,
and/or for delaying micturition.

## Revendications

1. Composés de formule I 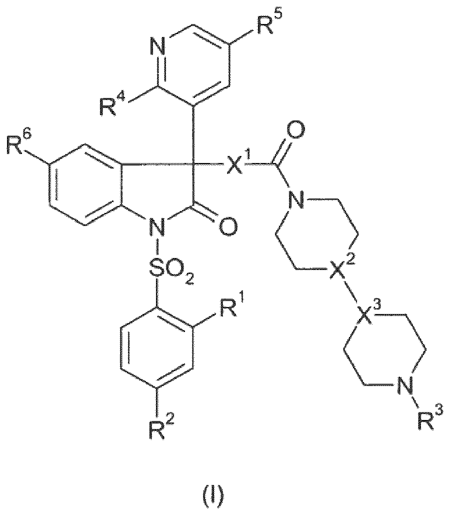 dans laquelle
R¹ représente hydrogène, méthoxy ou éthoxy ;
R² représente hydrogène ou méthoxy ;
R³ représente hydrogène, méthyle, éthyle, n-propyle ou isopropyle ;
R⁴ représente éthoxy ou isopropoxy ;
R⁵ représente H ou méthyle ;
R⁶ représente Cl ou F ;
X¹ représente O, NH ou CH₂ ; et
X² et X³ représentent N ou CH, à condition que X² et X³ ne représentent pas simultanément N ;
ainsi que leurs sels pharmaceutiquement compatibles.

2. Composés selon la revendication 1, dans lesquels R¹ représente hydrogène ou méthoxy.

3. Composés selon la revendication 1, dans lesquels R¹ et R² représentent méthoxy.

4. Composés selon l'une quelconque des revendications précédentes, dans lesquels R³ représente hydrogène, méthyle ou éthyle.

5. Composés selon l'une quelconque des revendications précédentes, dans lesquels R⁴ représente éthoxy et R⁵ représente H.

6. Composés selon l'une quelconque des revendications 1 à 4, dans lesquels R⁴ représente éthoxy et R⁵ représente méthyle.

7. Composés selon l'une quelconque des revendications 1 à 4, dans lesquels R⁴ représente isopropoxy et R⁵ représente H.

8. Composés selon l'une quelconque des revendications précédentes, dans lesquels R⁶ représente Cl.

9. Composés selon l'une quelconque des revendications 1 à 7, dans lesquels R⁶ représente F.

10. Composés selon l'une quelconque des revendications précédentes, dans lesquels X² représente N et X³ représente CH.

11. Composés selon l'une quelconque des revendications 1 à 9, dans lesquels X² représente CH et X³ représente N.

12. Composés selon l'une quelconque des revendications précédentes, dans lesquels
R¹ représente méthoxy ;
R² représente méthoxy ;
R³ représente méthyle ;
R⁴ représente éthoxy ;
R⁵ représente H ;
R⁶ représente Cl ;
X¹ représente NH ;
X² représente N ; et
X³ représente CH.

13. Composés selon l'une quelconque des revendications 1 à 11, dans lesquels
R¹ représente méthoxy ;
R² représente méthoxy ;
R³ représente méthyle ;
R⁴ représente éthoxy ;
R⁵ représente méthyle ;
R⁶ représente Cl ;
X¹ représente NH ;
X² représente N ; et
X³ représente CH.

14. Composés selon l'une quelconque des revendications 1 à 11, dans lesquels
R¹ représente méthoxy ;
R² représente méthoxy ;
R³ représente éthyle ;
R⁴ représente éthoxy ;
R⁵ représente H ;
R⁶ représente Cl ;
X¹ représente NH ;
X² représente N ; et
X³ représente CH.

15. Composés selon l'une quelconque des revendications 1 à 11, dans lesquels
R¹ représente méthoxy ;
R² représente méthoxy ;
R³ représente méthyle ;
R⁴ représente éthoxy ;
R⁵ représente H ;
R⁶ représente Cl ;
X¹ représente NH ;
X² représente CH ; et
X³ représente N.

16. Composés selon l'une quelconque des revendications précédentes, dans lesquels il s'agit de l'énantiomère (+) en une pureté énantiomérique d'au moins 90 % ee.

17. Composé selon l'une quelconque des revendications 1 à 15, dans lequel il s'agit du racémat.

18. Agent pharmaceutique, contenant au moins un composé de formule I selon la définition dans l'une quelconque des revendications précédentes et/ou au moins un de ses sels pharmaceutiquement compatibles et au moins un véhicule pharmaceutiquement compatible.

19. Utilisation de composés de formule I selon la définition dans l'une quelconque des revendications 1 à 17 ou de leurs sels pharmaceutiquement compatibles pour la fabrication d'un médicament pour le traitement et/ou la prophylaxie de maladies dépendantes de la vasopressine.

20. Utilisation selon la revendication 19 pour la fabrication d'un médicament pour le traitement et/ou la prophylaxie de maladies choisies parmi le diabète, la résistance à l'insuline, l'énurésie nocturne, l'incontinence, les maladies dans le cadre desquelles des troubles de la coagulation sanguine se produisent, l'hypertension, l'hypertension pulmonaire, l'insuffisance cardiaque, l'infarctus du myocarde, le spasme coronarien, l'angine instable, l'ACTP (angioplastie coronarienne transluminale percutanée), les ischémies du coeur, les troubles du système rénal, les oedèmes, le vasospasme rénal, la nécrose du cortex rénal, l'hyponatrémie, l'hypokaliémie, le syndrome de Schwartz-Bartter, les troubles du tractus gastrointestinal, le vasospasme gastrique, l'hépatocirrhose, l'ulcère de l'estomac et de l'intestin, les vomissements, les vomissements se produisant pendant la chimiothérapie, le mal des transports,
les troubles affectifs,
les troubles de l'anxiété, les troubles de l'anxiété liés au stress,
les troubles de la mémoire, la maladie d'Alzheimer,
les psychoses, les troubles psychotiques,
le syndrome de Cushing, les autres maladies liées au stress,
les troubles du sommeil,
les maladies dépressives, de préférence les troubles de l'humeur chez l'enfant,
les symptômes vasomoteurs, les dysfonctionnements thermorégulatoires,
les dépendances médiées par les drogues, les médicaments et/ou d'autres facteurs ; le stress causé par le sevrage d'un ou de plusieurs facteurs médiant la dépendance ; et/ou les rechutes induites par le stress dans les dépendances médiées par les drogues, les médicaments et/ou d'autres facteurs,
la schizophrénie et la psychose,
et/ou pour le retardement de la miction.
